# EUROPEAN PATENT SPECIFICATION

(11) **EP 2 613 829 B1**
(45) Date of publication and mention of the grant of the patent: **29.06.2022**
(21) Application number: 11757244.6
(22) Date of filing: 05.09.2011
(51) Int. Cl.: A61M 15/00, B65D 83/54, B65D 83/68, A61J 1/20, B05B 11/00, B65D 83/14

(54) **METERED-DOSE INHALER**
INHALIERGERÄT MIT ABGEMESSENER DOSIS
AÉROSOL-DOSEUR

(30) Priority: 06.09.2010 EP 10175430
(43) Date of publication of application: 17.07.2013
(73) Proprietor: Chiesi Farmaceutici S.p.A., 43122 Parma (IT)
(72) Inventor: BRAMBILLA, Gaetano, 43122 Parma (IT); LEWIS, David Andrew, 43122 Parma (IT); JOHNSON, Robert, 43122 Parma (IT); HOWLETT, David, 43122 Parma (IT)
(74) Representative: Adam, Holger
(86) International application number: PCT/EP2011/065303
(87) International publication number: WO 2012/032010

(56) References cited:
- WO-A1-2009/052563
- GB-A- 2 326 334
- US-A- 3 421 698
- US-A- 3 709 437
- US-A1- 2005 263 618
- US-A1- 2005 268 909
- US-A1- 2007 069 046
- US-A1- 2007 233 012
- US-A1- 2010 044 394
- GHASEM G NASR ANDREW J YULE LOTHAR BENDIG ED - NASR G G ET AL: "Chapter 2. Background on Sprays and Their Production", 1 January 2002 (2002-01-01), INDUSTRIAL SPRAYS AND ATOMIZATION : DESIGN, ANALYSIS AND APPLICATIONS, SPRINGER, LONDON, PAGE(S) 7 - 33, XP009195118, ISBN: 978-1-4471-3816-7

## Description

### FIELD OF THE INVENTION

The invention relates to a metered-dose inhaler.

### BACKGROUND OF THE INVENTION

Among the devices available to deliver medicaments to the lung, metered-dose inhalers (MDIs) are widely used. MDIs are aerosol delivery systems designed to deliver a medicament which may be formulated with a solvent, such as a compressed, low boiling point liquid gas propellant. MDIs are designed to meter a predetermined quantity of the medicament, completely dissolved (in solution) or suspended in the formulation and dispense the dose as an inhalable aerosol cloud or plume.

A conventional MDI includes an actuator and a canister. When the MDI is prepared for use, the canister is received in the actuator. The canister contains a formulation wherein the medicament is in solution or in suspension with a low boiling point propellant. The canister may be provided with a metering valve having a hollow valve stem for measuring discrete doses of the medicament formulation. When the canister is depressed into the actuator, a pre-determined dose of the medicament formulation is delivered from the canister. The dose of the medicament formulation is atomized at a nozzle orifice which may be disposed within the actuator. The dose is delivered from the MDI as an inhalable cloud or plume.

The traditional MDI design may impose restrictions on the active ingredients or formulations which can be delivered or on the delivery characteristics which can be attained. For illustration, it may not be possible to attain a desired particle size distribution or fine particle dose with arbitrary solvents, such as solvents which allow a high loading with an active ingredient. Vice versa, when using a particular propellant/solvent system, it may be difficult to attain a desired particle size distribution or fine particle dose. While the delivery characteristics may in some cases be influenced by an appropriate design of the nozzle orifice, this may not always allow the desired delivery characteristics to be obtained. Further, the traditional MDI design imposes restrictions on the co-administration of active ingredients or other excipients. For illustration, physical or chemical incompatibility may not allow different excipients or active ingredients to be formulated in an aerosol formulation which is contained in a container for extended time periods.

WO 92/16249 discloses an inhaler device that is able to house multiple removable canisters of medication and may also have extendable and retractable nozzles, spacer devices, a cover and assorted cap designs to ensure proper use and application of the medication. The inhaler device is configured to sequentially deliver medication from the multiple canisters.

WO 02/072183 A1 describes a dual-canister inhaler. The inhaler has a canister selection mechanism which allows one of two canisters to be selected, from which formulation will be dispensed upon actuation of the inhaler.

US 5,002,048 describes an inhalation device utilizing two or more aerosol containers. A housing has two receptacles to receive separate aerosol containers. The device is configured such that one of the aerosol containers is actuated by a patient for delivery of a dose of medication, such that different formulations are delivered in a sequential manner. Mixing of formulations is prevented by the inhalation device.

WO 03/061744 A1, WO 2004/011070 A1 and US 2005/268909 A1 respectively relate to a device comprising a first medicament container containing plural co-formulation compatible medicament components; first release means for releasing the contents of the first medicament container for delivery thereof; at least one or more medicament container, each containing at least one co-formulation incompatible medicament component; and at least one further release means for releasing the contents of each at least one further medicament container for delivery thereof.

WO 2009/052563 relates to an inhaler assembly for providing aerosol medications to animals, especially equines. The inhaler assembly comprises a canister holder for retaining at least two canisters, an outlet in communication with a spacer which is in communication with a mask through which a medication from at least one of the canisters is dispensed.

Further inhalation devices are described in US 2005/0263618 A1, US 2007/233012 A1, US 3,709,437, US 3,421,698, US 2007/069046 A1, and US 2010/044394 A1.

While inhalers such as the ones described in WO 02/072183 A1 or in US 5,002,048 allow one of plural different formulations to be selectively delivered, there is a continued need in the art for metered-dose inhalers which provide enhanced versatility in controlling delivery characteristics, such as the particle size distribution. There is also a continued need in the art for metered-dose inhalers which reduce the restrictions imposed on the formulator by the traditional MDI design.

In view of the above, there is a continued need in the art for metered-dose inhalers and methods which address some of the above needs.

### SUMMARY

These and other needs are addressed by a metered-dose inhaler as defined in claim 1. The dependent claims define embodiments.

The present invention relates to an metered-dose inhaler system that has at least two formulation reservoirs, each metered by a distinct valve system, which delivers the formulations through either a single orifice or separate orifices.

According to an aspect, the metered-dose inhaler comprises at least one vessel and an actuator for receiving the at least one vessel. The at least one vessel includes a first reservoir containing a first formulation and a second reservoir different from the first reservoir and containing a second formulation. The metered-dose inhaler is configured to be actuable when the at least one vessel is received by the actuator. The metered-dose inhaler, in the state in which the at least one vessel is received in the container, is configured to simultaneously deliver at least a first metered dose of the first formulation from the first reservoir and a second metered dose of the second formulation from the second reservoir upon actuation of the metered-dose inhaler.

The metered-dose inhaler allows formulations from independent reservoirs to be simultaneously delivered. A metered-dose inhaler having this configuration allows the delivery characteristics of one of the formulations to be modulated by mixing with the other one of the formulations. First and second formulations, or active agents or other excipients contained therein, which would give rise to stability problems when formulated together, may be co-administered using the metered-dose inhaler.

The metered-dose inhaler does not need to be fully assembled. For illustration, the metered-dose inhaler may be provided in the form of a kit including the actuator and the at least one vessel, in a state in which the at least one vessel is not yet fully assembled with the actuator. A patient may insert the at least one vessel into the actuator. In further embodiments, the metered-dose inhaler may be assembled, with the at least one vessel received in the actuator.

At least one of the first formulation and the second formulation may include an active ingredient. The first formulation and the second formulation may respectively be a solution formulation or a suspension formulation. The first formulation and the second formulation may be different from each other.

Both the first reservoir and the second reservoir are pressurized.

The metered-dose inhaler may comprise a first metering system for metering the first metered dose and a second metering system for metering the second metered dose. The first and second metering systems may be distinct. The first metering system may include a first metering valve. The second metering system may include a second metering valve.

The first metering system may meter between 1 µl and 100 µl per dose. The second metering system may meter between 1 µl and 100 µl per dose.

The first metering system may be configured to provide the first metered dose independently of the duration of actuation. The second metering system may be configured to provide the second metered dose independently of the duration of actuation. Thereby, consistent first doses and consistent second doses may be delivered upon repeated actuation of the metered-dose inhaler.

The metered-dose inhaler may comprise an actuating arrangement configured to effect simultaneous actuation of the first metering system and the second metering system upon actuation of the metered-dose inhaler, when the at least one vessel is received by the actuator. The actuating arrangement may have various configurations and may be formed integrally with other components of the metered-dose inhaler. For illustration, the actuating arrangement may include a joint valve stem of a first valve and of a second valve. The actuating arrangement may also include a rigid coupling between a first valve stem of a first valve and a second valve stem of a second valve. The actuating arrangement may also include a mechanical arrangement configured to effect a joint movement of separate vessels defining the first and second reservoirs.

The actuator may be configured such that, when the at least one vessel is received by the actuator, the first metered dose and the second metered dose are mixed prior to being delivered through a mouthpiece opening of the actuator upon actuation of the metered-dose inhaler. Thereby, a particle size distribution or fine particle dose may be modulated prior to the formulations being delivered to a patient.

The actuator may comprise a nozzle orifice for atomizing both the first metered dose and the second metered dose upon actuation of the metered-dose inhaler. This allows the first formulation and the second formulation to be mixed prior to atomization of the formulations.

The at least one vessel may have a valve stem for supplying both the first metered dose from the first reservoir and the second metered dose from the second reservoir. The actuator may have a seat for receiving the valve stem, the nozzle orifice being in communication with the seat for receiving the valve stem. This configuration allows the first and second metered doses to be mixed in the valve stem. The seat and nozzle orifice may be defined by an actuator block disposed within a housing of the actuator.

The at least one vessel may have a first valve stem for supplying the first metered dose and a second valve stem for supplying the second metered dose. The actuator may have a first seat for receiving the first valve stem and a second seat for receiving the second valve stem, the nozzle orifice being in communication with both the first seat and the second seat. This configuration allows the first and second metered doses to be mixed prior to atomization when the first and second metered doses are supplied through separate valve stems. The first and second seats and the nozzle orifice may be defined by an actuator block disposed within a housing of the actuator.

The actuator may comprise a first passageway for supplying the first metered dose to the nozzle orifice and a second passageway for supplying the second metered dose to the nozzle orifice. The first passageway may be linear. The second passageway may be linear.

The actuator may have a housing portion for housing the vessels, which housing portion has a longitudinal axis. The first passageway may be arranged at an angle relative to the longitudinal axis. The angle between a longitudinal axis of the first passageway and the longitudinal axis of the housing portion is a first orifice angle. The second passageway may be arranged at an angle relative to the longitudinal axis. The angle between a longitudinal axis of the second passageway and the longitudinal axis of the housing portion is a second orifice angle. The first orifice angle and the second orifice angle may respectively be included in the interval 0°-90°, in particular in the interval from 15° to 60°, in particular in the interval 20°-60°. The first orifice angle and the second orifice angle may be identical. Simultaneous delivery of the first and second metered doses is facilitated with an actuator having such a configuration.

The at least one vessel may have a first valve stem for supplying the first metered dose and a second valve stem for supplying the second metered dose. The actuator may define a first nozzle orifice, a second nozzle orifice separate from the first nozzle orifice, a first seat for receiving the first valve stem and a second seat for receiving the second valve stem. The first nozzle orifice may be in communication with the first seat and the second nozzle orifice may be in communication with the second seat. This configuration allows the first metered dose and the second metered dose to be atomized through separate first and second nozzle orifices. The atomized doses may be made to interact. The first and second seats and the first and second nozzle orifices may be defined by an actuator block disposed within a housing of the actuator. The actuator block may be configured such that the first nozzle orifice is not in communication with the second seat, and that the second nozzle orifice is not in communication with the first seat.

The first nozzle orifice and the second nozzle orifice may be arranged such that a longitudinal axis of the first nozzle orifice and a longitudinal axis of the second nozzle orifice are disposed at an angle relative to each other. The angle between the longitudinal axis of the first nozzle orifice and the longitudinal axis of the second nozzle orifice is an impingement angle. The impingement angle may be included in a range from 0° to 180°, in particular from 10° to 110°, in particular from 15° to 60°, with the ranges respectively including the range boundaries. This configuration allows the plume of one of the formulations to be directed towards the plume of the other formulation.

The at least one vessel may comprise a vessel having a first compartment and a second compartment. The first compartment may define the first reservoir, and the second compartment may define the second reservoir. Integration of the first reservoir and the second reservoir into one vessel enhances user comfort when assembling the metered-dose inhaler. The vessel may have outer dimensions identical to the ones of a canister used in conventional metered-dose inhalers.

The second compartment may be formed by a container disposed in an interior of the vessel. The container may be a canister having outer dimensions smaller than the inner dimensions of the vessel.

The vessel having the first and second compartments may be provided with one single valve stem through which both the first metered dose of the first formulation and the second metered dose of the second formulation are delivered. This configuration allows the first metered dose and the second metered dose to be mixed within the valve stem.

The vessel having the first and second compartments may be provided with a first valve stem for supplying the first metered dose and a second valve stem for supplying the second metered dose. The first valve stem and the second valve stem may be co-axially aligned. The first valve stem and the second valve stem may be rigidly joined together. This configuration allows the first metered dose and the second metered dose to be atomized through separate stem apertures. The atomized doses may interact with each other post atomization.

The at least one vessel may comprise a first vessel defining the first reservoir and a second vessel defining the second reservoir and formed separately from the first vessel. The first vessel may be formed as a first canister and the second vessel may be formed as a second canister. Each one of the canisters may be provided with a distinct metering system.

At least one of the first formulation and the second formulation is selected such that a particle size distribution, after atomization, of at least the other one of the first formulation and the second formulation is modulated by mixing the first metered dose and the second metered dose. Selecting one of the formulations such that the particle size distribution of the other one is modulated provides increased flexibility to the formulator.

At least one of the first formulation and the second formulation may be selected such that a fine particle dose of at least the other one of the first formulation and the second formulation after atomization is modulated by mixing the first metered dose and the second metered dose.

At least one of the first formulation and the second formulation may include a pharmaceutical agent, solvent, propellant or other excipient which would be incompatible with the other one of the first formulation and the second formulation, when formulated in the same container. Alternatively or additionally, at least one of the first formulation and the second formulation may include a pharmaceutical agent, solvent, propellant or other excipient which would be incompatible with a material of a canister, a canister interior coating, a valve or valve coating of the canister containing the other one of the first formulation and the second formulation. Incompatibility may result from chemical or physical incompatibility, which may give rise to unsatisfactory chemical or physical stability. Each one of the first and second reservoirs may have independent formulation solubilisers and/or stabilizers and/or packaging (can, can coating, valve material or coating) which would be incompatible in the same formulation.

The at least one vessel may include a third reservoir containing a third formulation.

The metered-dose inhaler may be configured, when the at least one vessel is received by the actuator, to simultaneously deliver the first metered dose of the first formulation, the second metered dose of the second formulation and a third metered dose of the third formulation from the third reservoir when the metered-dose inhaler is actuated. This allows three active pharmaceutical agents to be simultaneously delivered.

According to another aspect, a vessel for use in a metered-dose inhaler is provided.

The vessel has a first compartment containing a first formulation and a second compartment containing a second formulation. The vessel has a first metering system for metering a first dose of the first formulation and a second metering system for metering a second dose of the second formulation. The vessel has a hollow stem configured to simultaneously deliver the first dose of the first formulation and the second dose of the second formulation.

The vessel of the aspect may be used in combination with a metered-dose inhaler actuator to effect the simultaneous delivery of the first dose of the first formulation and the second dose of the second formulation through one nozzle orifice or through separate nozzle orifices. The first and second formulations may be mixed in the valve stem or at the nozzle orifice.

According to another aspect, a metered-dose inhaler actuator is provided. The actuator includes an actuator block defining a first seat for receiving a first valve stem and a second seat for receiving a second valve stem. The actuator block further defines one nozzle orifice. The actuator block includes channels communicating the first seat with a nozzle orifice of the nozzle orifice, and channels communicating the second seat with the nozzle orifice.

The actuator of this aspect allows a first formulation and a second formulation to be delivered from separate vessels.

In the actuator, one nozzle orifice may be in communication with both the first seat and the second seat.

Various effects and advantages are attained by devices and methods of embodiments. For illustration, in embodiments, a multi-reservoir system that combines formulations either pre- or post-exit orifice provides the ability to focus upon solubility and stability during formulation. The particle size distribution (PSD) and efficiency of a formulation can be modulated and/or enhanced by atomisation with a second, or optionally also third etc., formulation. In embodiments, a multi-reservoir system that combines formulations either pre- or post-exit orifice allows non-compatible excipients can be mixed at the time of atomization. In embodiments, a multi-reservoir system that combines formulations either pre- or post-exit orifice allows the consistency between the PSDs of mixed formulations to be controlled by design and selection of the mixing process (valve/can/actuator). This allows PSDs to be designed to match each other, range between the two (or more) initial formulations, or remain separate. According to various embodiments, the same or different metering volumes may be used for the different formulations. A variety of nozzle positions may be used in embodiments, which may be accurately selected to attain a desired nozzle positioning.

### BRIEF DESCRIPTION OF THE DRAWINGS

Fig. 1 is a schematic cross-sectional view of a metered-dose inhaler.
Fig. 2 is a schematic cross-sectional view of a metered-dose inhaler.
Fig. 3 is a schematic cross-sectional view of a two-compartment vessel of a metered-dose inhaler in a non-actuated state.
Fig. 4 is a schematic cross-sectional view of the vessel of Fig. 3 in an actuated state.
Fig. 5 is a schematic cross-sectional view of a two-compartment vessel of a metered-dose inhaler in a non-actuated state.
Fig. 6 is a schematic cross-sectional view of the vessel of Fig. 5 in an actuated state.
Fig. 7 is a cross-sectional view of a two-compartment vessel of a metered-dose inhaler in a non-actuated state.
Fig. 8 illustrates a sealing arrangement for an inner container of the vessel of Fig. 7.
Fig. 9 is a partially broken away cross-sectional view of the two-compartment vessel of Fig. 7 in a non-actuated state (left) and an actuated state (right).
Figs. 10-13 illustrate valve stem and nozzle orifice configurations of metered-dose inhalers.
Fig. 14A is a schematic cross-sectional view of a metered-dose inhaler according to an embodiment.
Fig. 14B shows an exploded view and plan views of a metered-dose inhaler according to an embodiment.
Fig. 14C shows a cross-sectional view of a guide member of the metered-dose inhaler of Fig. 14B.
Fig. 15A is a schematic cross-sectional view of a metered-dose inhaler according to a comparative example.
Fig. 15B is a view illustrating a cross-section through an actuator having plural nozzle orifices.
Fig. 16A illustrates an arrangement of containers in a metered-dose inhaler according to an embodiment.
Fig. 16B shows a metered-dose inhaler according to an embodiment.
Fig. 17A represents a cross-sectional view through a nozzle block having a single orifice and Fig. 17B represents a cross-sectional view through a nozzle block having two separate orifices.
Figs. 18 is a graph representing delivery characteristics for the simultaneous delivery of two formulations with different particle size distributions through one nozzle orifice of diameter 0.22 mm.
Fig. 19 is a graph representing delivery characteristics for the simultaneous delivery of a formoterol formulation and placebo formulation with different particle size distributions through one nozzle orifice of diameter 0.22 mm.
Fig. 20 is a graph representing delivery characteristics for the simultaneous delivery of a formoterol formulation and an incompatible budesonide formulation through one nozzle orifice of diameter 0.22 mm.
Fig. 21 is a graph representing delivery characteristics for the simultaneous delivery of a beclometasone dipropionate (BDP) (50µg/25µL) formulation and budesonide (50µg/100µL) formulation with a low volatility component through one nozzle orifice of diameter 0.22 mm.
Fig. 22 is a graph representing delivery characteristics for the simultaneous delivery of a BDP (50µg/50µL) formulation and budesonide (50µg/50µL) formulation with a low volatility component through one nozzle orifice of diameter 0.22 mm.
Fig. 23 is a graph representing delivery characteristics for the simultaneous delivery of a BDP (50µg/100µL) formulation and budesonide (50µg/25µL) formulation with a low volatility component through one nozzle orifice of diameter 0.22 mm.
Fig. 24 is a graph representing delivery characteristics for the simultaneous delivery of BDP (50µg/25µL) formulation and budesonide (50µg/25µL) formulation, both including a low volatility component, through separate nozzle orifices of diameter 0.22 mm.
Fig. 25 is a graph representing delivery characteristics for the simultaneous delivery of a BDP (50µg/25µL) formulation and budesonide (50µg/25µL) formulation with a low volatility component through separate nozzle orifices of diameter 0.22 mm.
Fig. 26 is a side view image of first and second plumes simultaneously delivered through separate nozzle orifices.
Fig. 27 shows images representing the plume cross section at various distances from the nozzle orifices.
Fig. 28 is a graph representing delivery characteristics for the simultaneous delivery of BDP (50µg/25µL) formulation and budesonide (50µg/25µL) formulation through one nozzle orifice having a diameter of 0.30mm.
Fig. 29 is a graph representing Andersen Cascade Impactor (ACI) drug deposition for delivery of a BDP (100µg/25µl), 26% w/w ethanol formulation and of a HFA 134a formulation through a single nozzle orifice of diameter 0.30mm.
Fig. 30 is a graph representing delivery characteristics for the simultaneous delivery of BDP (50µg/25µL) formulation and budesonide (50µg/100µL) formulation through one nozzle orifice of diameter 0.30mm.
Fig. 31 is a graph representing delivery characteristics for the simultaneous delivery of BDP (50µg/100µL) formulation and budesonide (50µg/25µL) formulation through one nozzle orifice of diameter 0.30mm.
Fig. 32 is a graph representing Andersen Cascade Impactor (ACI) drug deposition for delivery of a BDP formulation and a budesonide formulation through a dual orifice configuration.
Fig. 33 is a graph representing delivery characteristics for the simultaneous delivery of two formulations through a single nozzle orifice and through a dual orifice configuration.
Fig. 34 is a graph representing delivery characteristics of a BDP formulation when delivered simultaneously with a Salbutamol Sulphate formulation through a dual orifice configuration.
Fig. 35 is a graph representing delivery characteristics of a Salbutamol Sulphate formulation when delivered simultaneously with a BDP formulation through a dual orifice configuration.
Fig. 36 is a graph representing delivery characteristics of a BDP formulation and Salbutamol Sulphate formulation when delivered simultaneously through a single orifice configuration.
Fig. 37 is a graph representing delivery characteristics when a combination product is delivered simultaneously with another formulation through a single orifice configuration.
Fig. 38 is a graph representing delivery characteristics for a dual reservoir system having an actuator with a single orifice (as shown in Fig. 14B), with one reservoir containing a BDP/Formoterol combination formulation and the other reservoir containing a glycopyrronium bromide formulation.
Fig. 39 is a graph representing delivery characteristics for a triple reservoir system having an actuator with a single orifice (as shown in Fig. 17B), with a first reservoir containing a BDP/Formoterol combination formulation, a second reservoir containing a glycopyrronium bromide formulation, and a third reservoir containing a budesonide formulation.

### DETAILED DESCRIPTION OF EMBODIMENTS

Exemplary embodiments of the invention will now be described with reference to the drawings. The features of the embodiments may be combined with each other unless specifically stated otherwise.

According to exemplary embodiments, a metered-dose inhaler (MDI) is provided which is configured as a pressurized MDI (pMDI). The MDI includes an actuator and at least one vessel. The at least one vessel includes a first reservoir and a second reservoir different from the first reservoir. When the at least one vessel is assembled with the actuator, the MDI is configured to simultaneously deliver at least a first metered dose of a first formulation from the first reservoir and a second metered dose of a second formulation from the second reservoir, as will be described in more detail below.

At least one of the first formulation and the second formulation may include at least one active ingredient in a propellant/solvent system and, optionally, further excipients. According to exemplary embodiments, both the first formulation and the second formulation respectively include at least one active ingredient. According to exemplary embodiments, at least one of the first formulation and the second formulation may not include an active ingredient.

MDIs according to embodiments allow two or more aerosol components to be mixed to form a common aerosol. The intimate mixing may occur pre-orifice or post-orifice, to generate a new aerosol that may be tailored using the MDI hardware, which includes the actuator, as well as the formulation(s).

Both the first formulation and the second formulation may be pressurized. To this end, any one of a variety of propellants may be utilized. For illustration rather than limitation, propellants known in the art which may be utilized in the first reservoir and/or second reservoir in MDIs according to embodiments include tetrafluorethane (HFC134a), tetrafluorethane (P-134a), heptafluoropropane (P-227), combinations thereof, or any other suitable propellant. The skilled person will appreciate that such suitable propellants are readily available. For illustration rather than limitation, further examples of propellants are described in "Remington: The Science and Practice of Pharmacy", Lippincott Williams & Wilkins, 21st edition (2005), pp. 1012 et seq.

The first reservoir containing the first formulation may be formed from a rigid material, in particular from a metal material. The first reservoir may be an aluminum, aluminum alloy or stainless steel canister. Similarly, the second reservoir containing the second formulation may be formed from a rigid material, in particular from a metal material. The second reservoir may be an aluminum, aluminum alloy or stainless steel canister. The outer boundaries of the first reservoir and of the second reservoir may respectively be formed such that they do not deform as doses are repeatedly dispensed therefrom. The canister defining the first reservoir and the canister defining the second reservoir may be separate from each other or may be combined in one vessel, as will be described in more detail below. The canister defining the first reservoir and/or the canister defining the second reservoir may have part or all of their internal surfaces made in anodized aluminum or lined by an inert coating material. If one of the canisters is arranged in the interior of the other canister, the one canister may have an anodized aluminum external surface or may have an inert coating on its exterior surface.

The first reservoir may be provided with a first metering valve, and the second reservoir may be provided with a second metering valve. The first/second metering valve may be configured to deliver a measured amount of the first/second formulation. The first/second metering valve may be configured such that the delivered dose amount is reproducible. The first/second metering valve may be configured for inverted use or for upright use. When configured for upright use, the respective metering valve may be provided with a dip tube. The dip tube may be dimensioned as a capillary dip tube. The first and second metering valves may have a construction based upon the construction of conventional metering valves used in conventional pMDIs. In particular, the first/second metering valve may include a first/second metering chamber. The size of the first/second metering chamber may be varied in accordance with the respective application, so that a desired first dose and second dose are delivered in use. The first/second valve may respectively include a metering gasket and a stem gasket. In a first position, which may be the rest position of the first/second valve, the stem gasket will form a seal which prevents formulation to flow between the first/second metering chamber and a valve stem, while the metering gasket may allow formulation to flow between the first/second metering chamber and the first/second reservoir. In a second position, which may be the actuated position of the first/second valve, the stem gasket will allow formulation to flow from the first/second metering chamber to a valve stem. The metering gasket may then form a seal which prohibits formulation from flowing between the first/second metering chamber and the first/second reservoir while the first/second valve is being actuated. In this manner, a consistent amount of the first and second formulation may be delivered.

According to embodiments, the MDI may be configured such that first and second metering valves are simultaneously actuated upon actuation of the MDI. As will be described in more detail with reference to the drawings, this may be attained in various ways. For illustration, the first metering valve and the second metering valve may be coupled to each other so as to effect simultaneous actuation of the first metering valve and the second metering valve.

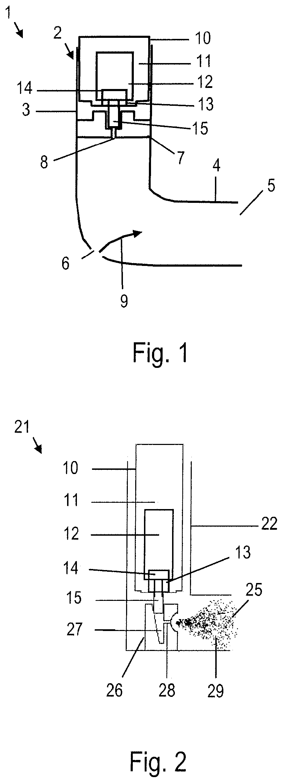

Fig. 1 is a schematic cross-sectional view of a metered-dose inhaler 1. The cross-sectional view is taken along the center symmetry plane of the MDI.

The MDI 1 includes an actuator 2 having a canister receiving portion 3 and a mouthpiece portion 4. Atomized formulations are delivered through a mouthpiece opening 5 in use of the MDI 1. An air inlet opening 6 is formed in the outer shell of the actuator 2 to allow air 9 to be drawn into the actuator housing through the inspiratory effort of a patient. An actuator block 7, which serves as nozzle block having a valve stem seat for receiving a valve stem 15, is disposed within the actuator housing. One nozzle orifice or plural nozzle orifices 8 are formed in the actuator block 7 for atomizing formulations upon actuation of the MDI 1.

The MDI 1 further includes a vessel 10. When the MDI 1 is ready for use, the vessel 10 is received by the actuator 2, and a valve stem of the vessel 10 is received in a seat formed in the actuator block 7. The interior of the hollow valve stem is in communication with the nozzle orifice in this state. The MDI 1 may be provided to a patient in a state in which the vessel 10 is not yet received in the actuator 2. The patient may then prepare a dose delivery by inserting the vessel 10 into the actuator 2. The vessel 10 may be removably received in the actuator 2, so as to allow washing of the actuator 2 after dose delivery.

The vessel 10 has a first reservoir 11 containing a first formulation and a second reservoir 12 containing a second formulation. At least one of the first formulation and the second formulation may contain an active ingredient. In some examples, both the first and the second formulations respectively contain an active ingredient. In further examples, at least one of the first and second formulations does not contain an active ingredient. The first and second formulations may be different from each other. The formulation(s) which contain(s) at least one active ingredient may be an aerosol suspension formulation or an aerosol solution formulation. The formulation(s) may include the at least one active ingredient in a propellant/solvent system and may optionally contain further excipients.

The vessel 10 has an outer canister defining an outer shell of the first reservoir 11 and an inner canister defining the second reservoir 12. The inner canister is completely enclosed by the outer canister. The inner and outer canisters are formed from a rigid material. The inner and outer canisters do not change their shapes when the MDI 1 is repeatedly actuated.

The MDI 1 has a first metering valve 13 and a second metering valve 14. The first and second metering valves may have different valve stems or may share one valve stem. The metering valves 13 and 14 may be integrated into the vessel 10. A valve stem 15 extends from the vessel 10 and may be received in a valve stem seat formed in the actuator block 7.

The first metering valve 13 meters a first dose of the first formulation, which is delivered upon actuation of the MDI 1. The second metering valve 14 meters a second dose of the second formulation, which is delivered upon actuation of the MDI 1. The metering valves 13 and 14 are non-continuous valves which respectively provide predetermined volumes of the respective formulation. The metering valves 13 and 14 are configured to reproducibly supply the pre-determined first and second doses. The first dose of the first formulation and the second dose of the second formulation may be equal in size or may have different sizes as function of a volume of the respective metering chamber.

The MDI 1 is configured such that the first metered dose and the second metered dose are simultaneously delivered, so that the first and second doses can be made to interact within the actuator housing. The MDI 1 may be configured such that the first metered dose and the second metered dose are mixed before atomization. The MDI 1 may be configured such that the first and second metered doses are mixed at the nozzle orifice 8. For illustration, the vessel 10 may be configured such that the first and second metered doses are mixed in the valve stem 15 upon actuation of the MDI 1 and are supplied to the nozzle orifice 8. The vessel 10 and actuator 2 may also be configured such that the first and second metered doses are separately supplied to the nozzle orifice 8 and are mixed in the nozzle orifice 8 prior to atomization.

Alternatively, the MDI 1 may be configured such that the first metered dose and the second metered dose interact after being delivered through separate nozzle orifices.

The MDI 1 may include an actuating arrangement configured to ensure simultaneous delivery of the first metered dose and the second metered dose. The arrangement may be integrally formed with other components of the MDI, such as with the first and second valves. For illustration, simultaneous delivery of the first and second metered doses may be attained by appropriately configuring the first metering valve and the second metering valve, so that the first and second metering valves are actuated simultaneously. The first and second metering valves may be configured such that, upon actuation of the MDI 1, a fluid communication is established with a hollow interior of the valve stem 15, so that both the first metered dose and the second metered dose are delivered from the first and second reservoirs, respectively, through the valve stem 15.

In use of the MDI 1, the patient may for example depress the container 10 into the actuator 2 to effect actuation. Other actuation mechanisms may be utilized. The first metered dose of the first formulation and the second metered dose of the second formulation are delivered through the nozzle orifice 8. The first and second metered doses may be mixed before atomization, such as in the valve stem 15 or in the nozzle orifice 8. Respirable particles in the spray cloud are entrained in the air flow 9. The mixed doses of the first and second formulation are delivered through the mouthpiece opening 5 of the MDI 1.

By appropriately selecting the formulations, the particle size distribution of at least one of the first and second formulations may be influenced by mixing with the other one of the first and second formulations. This provides enhanced control over the delivery characteristics.

The MDI 1 may allow the formulator to focus on the solubility and stability of the first and second formulations during formulation. The particle size distribution may be influenced by atomizing the first and second formulations together. The particle size distribution of at least one of the first and second formulations may be controlled by mixing with the other one of the first and second formulations, at the time of delivery.

The MDI 1 also allows non-compatible excipients to be stored in independent reservoirs and to be mixed in the actuator prior to delivery to the patient.

The particle size distributions of the first and second formulations may be controlled via the mixing process. Control over the particle size distributions can be attained by appropriately selecting the first dose and the second dose, the configuration of one or plural nozzle orifices in the actuator, and/or the actuator geometry. For illustration, in implementations, the particle size distributions of the first and second formulations can be designed to match each other. The particle size distributions of the first and second formulations can be designed to range between the particle size distributions which would be obtained for atomizing the first formulation through a single actuator and the particle size distributions which would be obtained for atomizing the second formulation through a single actuator.

While the MDI 1 of Fig. 1 has a design in which a longitudinal axis of the nozzle orifice 8 is aligned with a longitudinal axis of the valve stem 15, other numbers and arrangements of the nozzle orifice may be implemented in further examples. For illustration, the nozzle orifice may be arranged such that its longitudinal axis is disposed at an angle, for example at 90° or at more than 90°, relative to the longitudinal axis of the valve stem 15, when the vessel 10 is received in the actuator 2. Plural separate nozzle orifices may be provided. The longitudinal axes of the plural nozzle orifices may be parallel, or may be disposed at an angle relative to each other.

Fig. 2 is a schematic cross-sectional view of an MDI 21 according to another example. The cross-sectional view is taken along the center symmetry plane of the MDI. Elements or features which correspond, with regard to their configuration and/or function, to elements or features of the MDI 1 of Fig. 1 are designated by the same reference numerals.

The MDI 21 has a two-compartment vessel 10 and an actuator 22. The actuator 22 has an actuator block 26, in which the valve stem 15 is received when the vessel 10 is inserted into the actuator 22. A first metered dose of the first formulation and a second metered dose of the second formulation are supplied to a nozzle orifice 28 via an internal chamber 27 upon actuation of the MDI 21. The first and second metered doses are mixed and are atomized. The atomized mixed formulations are delivered as a spray cloud 29. In the actuator 22, the nozzle orifice 28 at which the first and second formulations are mixed, is disposed at an angle relative to the longitudinal axis of the valve stem 15 when the vessel 10 is received in the actuator 22.

In the MDIs 1 and 21, the first and second formulations may be mixed and delivered through one nozzle orifice. The outer canister, which forms the outer shell of the vessel 10, may have dimensions identical to canisters used in conventional MDIs. This allows actuators of conventional dimensions and/or designs to be used in association with the dual-compartment vessel.

The dual-compartment vessel and the valve assembly for use in an MDI 1, 21 may have various configurations. For illustration, the first and second metering valves may share one valve stem. In further examples, the first metering valve may have a first valve stem through which the first metered dose is supplied to a nozzle orifice, and the second metering valve may have a second valve stem through which the second metered dose is supplied to a nozzle orifice. The first and second valve stems may be rigidly coupled.

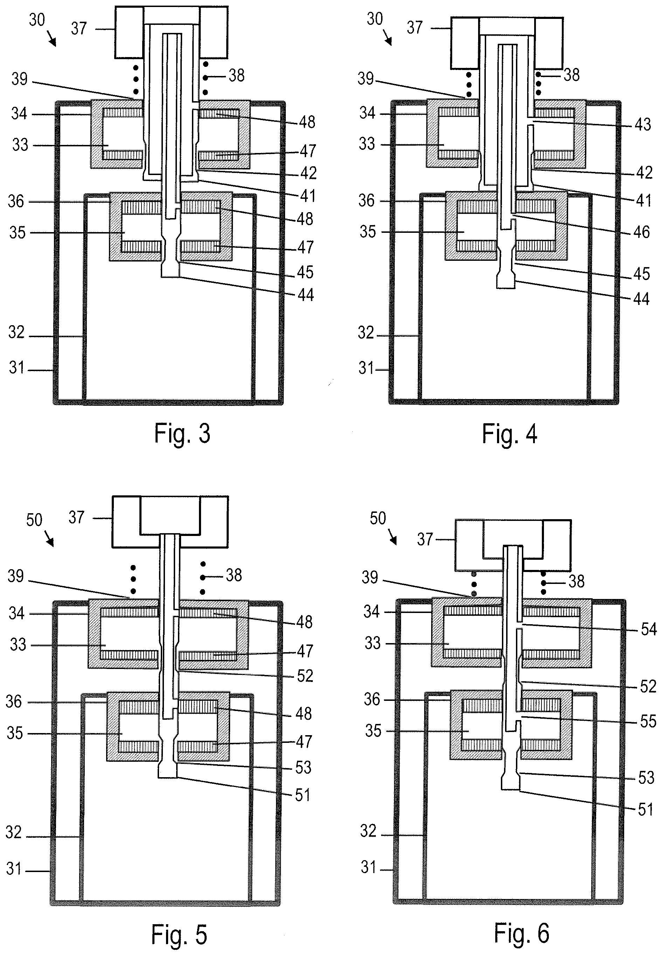

Configurations of dual-compartment vessels and valve assemblies for an MDI according to various examples will be explained in more detail with reference to Figs. 3-9.

Fig. 3 is a schematic cross-sectional view of a two-compartment vessel 30 and valve assembly in a non-actuated rest state. Fig. 4 is a schematic cross-sectional view of the two-compartment vessel 30 and valve assembly in an actuated state.

The vessel 30 has an outer canister 31 and an inner canister 32. The inner canister 32 separates a first formulation contained in the space defined between the outer canister 31 and the inner canister 32 from a second formulation contained in the interior of the inner canister 32. The outer canister 31 and inner canister 32 are respectively formed so as to be rigid. A first pressurized aerosol formulations is contained in the space defined between the outer canister 31 and the inner canister 32. A second pressurized aerosol formulations is contained in the second canister 32.

A first metering valve and a second metering valve are formed integrally with the vessel 30. The first metering valve schematically indicated at 34 defines a first metering chamber 33. The second metering valve schematically indicated at 36 defines a second metering chamber 35. A first valve stem 41 and a second valve stem 44 are provided. The first valve stem 41 and the second valve stem 44 are provided in a coaxial configuration. The first valve stem 41 and the second valve stem 44 are attached to each other such that they are axially fixed, i.e., such that the first valve stem 41 and second valve stem 44 are forced to be jointly displaced in an axial direction. The first and second valve stems 41 and 44 may be rigidly coupled to each other. The combination of the first and second valve stems 41 and 44 may be considered to form a joint valve stem having a split configuration, in which different formulations move in different portions of the joint valve stem.

The first valve stem 41 includes one or plural recessed portions 42 or other concavities. The recessed portions 42 are disposed such that the space interior of the first canister 31 and exterior of the first metering valve 34 is in fluid communication with the first metering chamber 33 when the valve is in the non-actuated state. The non-actuated rest state, into which the valve stem 41 is biased by a biasing member, e.g. spring 38, is illustrated in Fig. 3. The recessed portions 42, which may be formed as slots, allow the first formulation to flow between the first reservoir and the first chamber 33, past a metering gasket member 47 provided in the first metering valve 34. The first formulation is allowed to flow in/out of the chamber until the point of actuation. The recessed portions 42 are further disposed such that the fluid communication is blocked between the first reservoir and the first metering chamber 33 when the first valve is actuated by displacement of the first valve stem 41. Upon actuation, the first metering chamber 33 is isolated from the first reservoir by the metering gasket. The actuated state which is attained by relative displacement of the first valve stem 41 is illustrated in Fig. 4.

The first valve stem 41 further includes one or plural openings 43. The opening 43 is disposed such that the first metering chamber 33 is not in fluid communication with the interior of the first valve stem 41 when the valve is in the non-actuated state, as illustrated in Fig. 3. In the non-actuated state, a seal may be formed which prevents the first formulation from moving from the first metering chamber 33 into the interior of the first valve stem 41. To this end, a stem gasket member 48 may be provided in the first metering valve 34. The opening 43 is further disposed such that the first metering chamber 33 is in fluid communication with the interior of the first valve stem 41 when the valve is in the actuated state, as illustrated in Fig. 4. Upon actuation, the first formulation moves from the chamber 33 into the valve stem 41 via opening 43.

The second valve stem 44 has a similar configuration. The second valve stem 44 includes one or plural recessed portions 45 or concavities formed therein. The portions 45 are disposed such that the space interior of the second canister 32 is in fluid communication with the second metering chamber 35 when the valve is in the non-actuated state. The recessed portions 45, which may be formed as slots, allow the second formulation to flow between the second reservoir and the second metering chamber 35, past a metering gasket member 47 provided in the second metering valve 36. The second formulation is allowed to flow in/out of the chamber until the point of actuation. The recessed portions 45 are further disposed such that the fluid communication is blocked between the second reservoir and the second metering chamber 35 when the second valve is actuated by displacement of the second valve stem 44. Upon actuation, the second metering chamber 35 is isolated from the second reservoir by the metering gasket. The actuated state which is attained by relative displacement of the second valve stem 44 is illustrated in Fig. 4.

The second valve stem 44 further includes one or plural openings 46. The opening 46 is disposed such that the second metering chamber 35 is not in fluid communication with the interior of the second valve stem 44 when the valve is in the non-actuated state, as illustrated in Fig. 3. In the non-actuated state, a seal may be formed which prevents the second formulation from moving from the second metering chamber 35 into the interior of the second valve stem 44. To this end, a stem gasket member 48 may be provided in the second metering valve 36. The opening 46 is further disposed such that the second metering chamber 35 is in fluid communication with the interior of the second valve stem 44 when the valve is in the actuated state, as illustrated in Fig. 4. Upon actuation, the second formulation moves from the second metering chamber 35 into the valve stem 44 via opening 46.

The first and second valves are configured such that, upon actuation of the valves, the first and second metered doses are simultaneously delivered. To this end, the recessed portions 42 and 45, the openings 43 and 46 and the gasket members 46 and 47 may be arranged such that the first metering chamber 33 may be filled with the first formulation and the second metering chamber 35 may be filled with the second formulation in parallel and while the valves are not actuated. The recessed portions 42 and 45, the openings 43 and 46 and the gasket members 46 and 47 may further be arranged such that fluid communication between the first metering chamber 33 and the interior of the first valve stem 41 may be established at the same time, upon actuation of the valve, when the fluid communication between the second metering chamber 35 and the interior of the second valve stem 44 is established. The recessed portions 42 and 45, the openings 43 and 46 and the gasket members 46 and 47 may further be arranged such that fluid communication between the first reservoir and the first chamber 33 and between the second reservoir and the second chamber 35 may be interrupted at the same time, upon actuation of the valve.

In the vessel 30 and valve assembly, the biasing member, such as a spring 38, is provided externally of the outer canister 31. The actuator seat 37 holds the external spring 38 in place against a valve ferrule 39. The biasing member may be configured to bias the first valve into a rest position in which the first metering chamber 33 is not in fluid communication with the hollow interior of the valve stem, i.e., in which a seal isolates the first metering chamber 33 from the hollow interior of the valve stem. The biasing member may be configured to bias the second valve into a rest position in which the second metering chamber 35 is not in fluid communication with the hollow interior of the valve stem, i.e., in which a seal isolates the second metering chamber 35 from the hollow interior of the valve stem.

It will be appreciated that the biasing member 38, which biases the valve stem and thus the first valve and/or the second valve into a rest position, may be positioned externally of the outer canister 31. By contrast, conventional canisters for MDI usually employ a biasing member disposed internally of the canister. The arrangement with the biasing member positioned externally of the canister, which may be implemented in various examples described herein, has the effect that the first and second formulations will not come into contact with the biasing member 38. The risk of potential contamination with metal ions, which can cause formulation instability, may be reduced by positioning the biasing member 38 externally of the outer canister 31.

Fig. 5 is a schematic cross-sectional view of a two-compartment vessel 50 and valve assembly in a non-actuated state. Fig. 6 is a schematic cross-sectional view of the two-compartment vessel 50 and valve assembly in an actuated state. Elements or features which correspond, with regard to their configuration and/or function, to elements or features of the vessel and valve assembly of Figs. 3 and 4 are designated by the same reference numerals.

The vessel 50 has a first canister 31, a second canister 32, and first and second metering chambers 33 and 35, respectively, as explained with reference to Figs. 3 and 4. The valve assembly has a single valve stem 51. A biasing member 38, e.g. a spring, biases the valve stem 51 into the non-actuated position illustrated in Fig. 5. It will be appreciated that the biasing member 38 may be positioned externally of the outer canister 31, as shown in Fig. 5.

The valve stem 51 is configured such that the first formulation can flow between the first reservoir and the first chamber 33 and that the second formulation can flow between the second reservoir and the second chamber 35 when the valve is not actuated. The valve stem 51 is configured such that seals, or gaskets, prohibit the first formulation from flowing from the first chamber 33 into the valve stem 51 and prohibit the second formulation from flowing from the second chamber 35 into the valve stem 51 when the valves are not actuated. The valve stem 51 is further configured such that the first chamber 33 is isolated from the first reservoir and the second chamber 35 is isolated from the second reservoir when the valves are actuated. The actuated state is illustrated in Fig. 6. The valve stem 51 is configured such that the first formulation moves from the first chamber 33 into the valve stem 51 and that the second formulation moves from the second chamber 35 into the valve stem 51 when the valves are actuated by axial displacement of the valve stem 51.

The valve stem 51 has one or plural first recesses 52 which allow the first formulation to flow freely between the first reservoir and the first metering chamber 33, past a metering gasket member 47 provided in the first valve 34, in the rest state shown in Fig. 5. The valve stem 51 has one or plural second recesses 53 which allow the second formulation to flow freely between the second reservoir and the second metering chamber 35 past a metering gasket member 47 provided in the second valve 36, in the rest state shown in Fig. 5. The first recesses 52 and second recesses 53 are arranged such that the first chamber 33 is isolated from the first reservoir and the second chamber 35 is isolated from the second reservoir by axial displacement of the valve stem 51 upon actuation, as illustrated in Fig. 6.

The valve stem 51 has one or plural first openings 54 which are arranged such that, in the rest state shown in Fig. 5, a seal is formed which prevents the first formulation from moving from the first chamber 33 to the interior of the valve stem 51. To this end, a stem gasket member 48 may be provided in the first valve 34. The at least one first opening 54 is arranged so as to allow the first formulation to move from the first chamber 33 to the interior of the valve stem 51 in the actuated state, as shown in Fig. 6. The valve stem 51 has one or plural second openings 55 which are arranged such that, in the rest state shown in Fig. 5, a seal is formed which prevents the second formulation from moving from the second chamber 35 to the interior of the valve stem 51. To this end, a stem gasket member 48 may be provided in the second valve 36. The at least one second opening 55 is arranged so as to allow the second formulation to move from the second chamber 35 to the interior of the valve stem 51 in the actuated state.

When the first metering valve and the second metering valve have a valve stem in common, as schematically illustrated in Figs. 5 and 6, the valve stem 51 may be formed to have a simple construction.

Further, the first metering valve and the second metering valve have a valve stem in common, the first metered dose of the first formulation and the second metered dose of the second formulation may be mixed in the valve stem. Pre-atomization mixing can be attained.

The two-compartment vessels and associated valve assemblies explained with reference to Figs. 3-6 may be used to simultaneously deliver the first formulation and the second formulation. The formulations may be mixed pre-atomization, as in the two compartments vessel 50 with a common valve stem (Fig. 5 and 6), or post-atomization. Post-atomization mixing may for example be attained using a two compartments vessel having a first valve stem and a second valve stem, which may be combined as illustrated in Fig. 3 and 4.

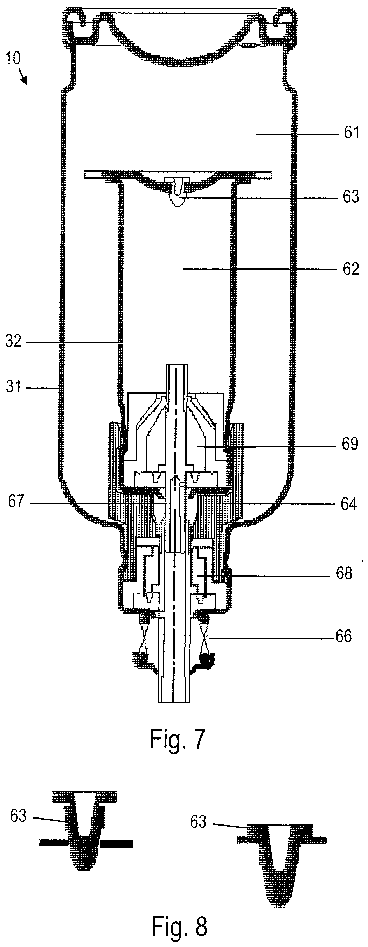

Various implementations of the two-compartment vessels and associated valve assemblies explained with reference to Figs. 3-6 may be realized. For illustration, an implementation of the single-stem vessel will be explained in more detail with reference to Figs. 7-9.

Fig. 7 shows a dual-compartment vessel 10 and associated valve assembly which may be used as vessel 10 in the MDIs of Figs. 1 and 2. Elements or features which correspond, with regard to their configuration and/or function, to elements or features of the vessel and valve assembly of Figs. 3-6 are designated by the same reference numerals.

The vessel 10 has an outer canister 31 and an inner canister 32. The outer canister 31 and the inner canister 32 may be formed so as to be rigid. A first reservoir 61 is defined in the interior of the outer canister 31 and exterior of the inner canister 32. A second reservoir 62 is formed in the interior of the inner canister 32. The first reservoir 61 contains a first formulation, and the second reservoir 62 contains a second formulation. The first and second formulations may be different from each other.

For illustration rather than limitation, the inner canister 32 may be formed from a 13 mm diameter can. The outer canister 31 can be formed from a standard 22 mm can. The 13 mm diameter used for the inner canister 32 can be fitted into the 22 mm can used for the outer canister.

The design of the combined valve stem allows another method for pressure filling the propellant, for example an HFA propellant or other propellant, to be employed. A plug 63 may be used to seal the base of the inner canister 32. The plug 63 may be disposed at a center of the base of the inner canister 32. As seen more clearly in Fig. 8, the plug 63 has engaging means for engaging the base of the inner canister 32. The engaging means are configured as a recess which receives an edge of the base defining the opening through which the plug 63 is inserted, as illustrated on the right in Fig. 8.

Mating engaging means may be provided in the interior of the outer canister 31 and on the exterior of the inner canister 32. For illustration, engaging projections 64 may be provided in the interior of the outer canister 31. The engaging projections engage with a structured outer surface of the inner canister 32.

A first valve for metering a first dose of the first formulation from the first reservoir and a second valve for metering a second dose of the second formulation from the second reservoir are provided. The first valve and the second valve have one valve stem 67, which is common to the first valve and the second valve. The valve stem 67 is biased towards a rest position by a bias means, such as spring 66. The bias means may be arranged externally of the outer canister 31.

The first valve and the second valve may have standard valve designs. Metering first and second doses may be performed using a mechanism generally corresponding to the one explained with reference to Figs. 5 and 6. In particular, the first and second valve may respectively include a metering chamber. The first and second valve may respectively be provided with a metering gasket and a stem gasket. The first and second valves may respectively be configured such that in a non-actuated rest state the metering chamber is in fluid communication with the respective reservoir while fluid communication between the metering chamber and an interior of the valve stem 67 is blocked. In an actuated state of the first or second valve, the associated metering chamber may respectively be isolated from the respective reservoir while the metering chamber may be in fluid communication with the hollow interior of the valve stem 67.

The valve assembly may include a first metering chamber 68 defined by the first valve. In the rest position, as illustrated in Fig. 7, the first formulation may flow between the first reservoir and the first metering chamber 68. A gasket may prevent flow between the first metering chamber 68 and the interior of the valve stem 67 at that time. When the valve stem 67 is axially displaced for actuation of the valves, the first metering chamber 68 is isolated from the first reservoir and the first formulation is allowed to move from the first metering chamber 68 to the interior cavity of the valve stem 67.

The valve assembly may include a second metering chamber 69 defined by the second valve. In the rest position, as illustrated in Fig. 7, the second formulation may flow between the second reservoir and the second metering chamber 69. A gasket may prevent flow between the second metering chamber 69 and the interior of the valve stem 67 at that time. When the valve stem 67 is axially displaced for actuation of the valves, the second metering chamber 69 is isolated from the second reservoir and the second formulation is allowed to move from the second metering chamber 69 to the interior cavity of the valve stem 67.

The valve stem 67, which is common to the first valve and the second valve, ensures that the first metered dose of the first formulation and the second metered dose of the second formulation are simultaneously delivered upon actuation of an MDI. The metered doses of the first and second formulations may then be mixed in the valve stem and/or at a nozzle orifice.

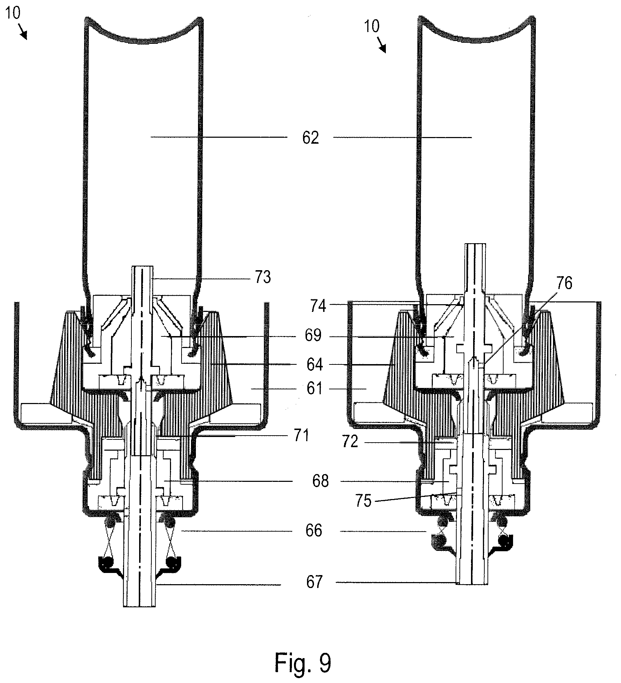

Fig. 9 shows a partially broken away view of the vessel 10 with associated valve assembly in the rest position (left) and in the actuated state (right).

In the rest position, recesses 71 in the valve stem 67, which may be formed as slots, allow the first formulation to flow from the first reservoir into the first metering chamber 68. Similarly, recesses 73 in the valve stem 67, which may be formed as slots, allow the second formulation to flow from the second reservoir into the second metering chamber 69.

In the actuated state shown on the right-hand side in Fig. 9, the valve stem 67 is axially displaced against the biasing force of the spring 66. Formation of a first seal 72 isolates the first metering chamber 68 from the first reservoir. Formation of a second seal 74 isolates the second metering chamber 69 from the second reservoir. In the actuated state, at least one first opening 75 in the valve stem 67 allows the first dose of the first formulation to move into the valve stem 67. At least one second opening 76 in the valve stem 67 allows the second dose of the second formulation to move into the valve stem 67.

In MDIs which include a dual-compartment vessel, which may be formed by an inner canister completely enclosed by an outer canister, mixing of the first and second formulations can occur within the valve stem, as explained with reference to Figs. 5-9, or at the actuator block defining an actuator seat for a valve stem, as explained with reference to Figs. 3 and 4. An actuator having a conventional design with one nozzle orifice defined in the actuator block may be used when mixing occurs in the valve stem or at the nozzle orifice.

According to some examples, the actuator may be provided with an actuator block having one or plural nozzle orifices. The number and arrangement of the nozzle orifices may be selected in accordance with desired actuator characteristics. Actuators defining a plurality of nozzle orifices may be used for applications in which it is desired to implement post-atomization mixing. To this end, the nozzle orifices may be configured such that spray clouds of the first and second formulation are made to impinge on each other upon actuation of the MDI.

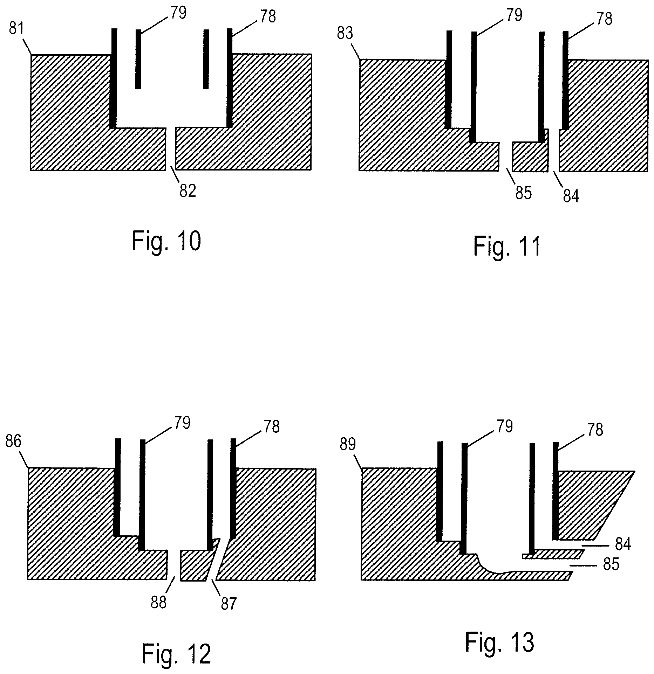

With reference to Figs. 10-13, configurations of an actuator block defining one or plural nozzle orifices will be explained. An actuator for receiving a dual-compartment vessel, such as the actuator 2 of the MDI 1 described with reference to Fig. 1 or the actuator 22 of the MDI 21 described with reference to Fig. 2, may define any one of the nozzle configurations schematically illustrated in Figs. 10-13. Still further nozzle configurations may be implemented in yet other examples.

For better understanding, end portions of an outer valve stem 78 and an inner valve stem 79 are also shown. The outer and inner valve stems are aligned co-axially and may be rigidly joined to each other, as explained with reference to Figs. 3 and 4.

Fig. 10 shows a cross sectional view of an actuator block 81. The actuator block 81 defines an actuator seat in which the outer and inner valve stems are received when the vessel is received by the actuator. The actuator block 81 defines one orifice 82. The orifice 82 is in fluid communication with the actuator seat. The orifice 82 may have a cylindrical shape. Other orifice shapes may be used. When the outer and inner valve stems are received in the actuator seat, the orifice 82 is in fluid communication with both the hollow interior of the inner valve stem 79 through which a second formulation is supplied and with the hollow space through which a first formulation is supplied and which is enclosed by the inner face of the outer valve stem 78 and the outer face of the inner valve stem 79.

The inner valve stem 79 containing the second formulation from a second reservoir meets the outer stem 78 containing the first formulation from a first reservoir at the actuator block 81. The formulations from the independent reservoirs are mixed at the actuator block and the mixed formulations atomize through the single orifice 82.

Figs. 11-13 illustrate other actuator block designs in which the first formulation is atomized at a first orifice and the second formulation is atomized at a second orifice different from the first orifice. When the MDI is actuated, the first formulation from the first reservoir moves through one of the inner and outer valve stems and atomizes at a first orifice, while the second formulation from the second reservoir moves through the other one of the inner and outer valve stems and atomizes at a second orifice.

Fig. 11 shows a cross sectional view of an actuator block 83. The actuator block 83 defines receptacles in which the outer and inner valve stems are received when the medicament-containing vessel is received by the actuator. The actuator block 83 defines two orifices 84 and 85. A first orifice 84 may be in fluid communication with the portion of the actuator seat in which the outer valve stem 78 is received. A second orifice 85 may be in fluid communication with the central portion of the actuator seat in which the inner valve stem 79 is received. The orifices 84 and 85 may respectively have a cylindrical shape. One or both of the orifices 84 and 85 may also be provided with shape other than cylindrical. The first orifice 84 and second orifice 85 are spaced from each other. The longitudinal axes of the orifices 84 and 85 are parallel. The longitudinal axes of the orifices 84 and 85 are aligned with the longitudinal axis of the actuator seats.

When the outer and inner valve stems are received in the actuator seats, the first orifice 84 is in fluid communication with the hollow space through which a first formulation is supplied and which is enclosed by the inner face of the outer valve stem 78 and the outer face of the inner valve stem 79. The second orifice 85 is in fluid communication with the hollow interior of the inner valve stem 79 through which a second formulation is supplied. Upon actuation of the MDI, the first formulation moves through the outer valve stem 78 and atomizes at the first orifice 84. The second formulation moves through the inner valve stem 79 and atomizes at the second orifice 85.

The construction of the nozzle block as illustrated in Fig. 11 allows the first formulation and the second formulation to interact after atomisation at the orifices 84 and 85, respectively. The particle size distribution and/or fine particle dose of a formulation may be influenced even by post-atomization mixing with another formulation.

When at least two orifices are defined by an actuator block, the longitudinal axes of the orifices may be disposed at an angle relative to each other, i.e., the orifices may be arranged such that the orifices are non-parallel. The first and second orifices may be arranged to form cross-flow orifice paths, in which formulation from one of the first and second reservoirs is atomized at one of the orifices, resulting in a spray pattern that causes flow interaction with the plume produced from atomization of the formulation from the other reservoir at the other orifice. Thereby, a modification of the combined plume geometry, a direction of the combined plume, or modification of the final aerosolised product may be realized.

In specific examples, the orifice spacing and an impingement angle may be set so as to yield aerosol plume intersection distance from 2.5cm to 10cm from the actuator. In particular an impingement angle between the orifices may be set to have a value included in the range from 15 to 60°.

Fig. 12 shows a cross sectional view of an actuator block 85 having a orifice design in which the longitudinal axes of the orifices are disposed at an angle relative to each other.

The actuator block 86 defines actuator seats in which the outer and inner valve stems are received when the medicament-containing vessel is received by the actuator. The actuator block 86 defines two orifices 87 and 88. A first orifice 87 may be in fluid communication with the portion of the actuator seat in which the outer valve stem 78 is received. A second orifice 88 may be in fluid communication with the central portion of the actuator seat in which the inner valve stem 79 is received. The orifices 87 and 88 may respectively have a cylindrical shape. One or both of the orifices 87 and 88 may also be provided with shape other than cylindrical. The first orifice 87 and second orifice 88 are spaced from each other. The longitudinal axes of the orifices 87 and 88 are disposed at an angle relative to each other. The longitudinal axis of one of the orifices 87 and 88 may be aligned with the longitudinal axis of the actuator seats.

When the outer and inner valve stems are received in the actuator seats, the first orifice 87 is in fluid communication with the hollow space through which a first formulation is supplied and which is enclosed by the inner face of the outer valve stem 78 and the outer face of the inner valve stem 79. The second orifice 88 is in fluid communication with the hollow interior of the inner valve stem 79 through which a second formulation is supplied. Upon actuation, the first formulation moves through the outer valve stem 78, passes the first orifice 87, and atomizes at the first orifice 87. The second formulation moves through the inner valve stem 79, passes the second orifice 88, and atomizes at the second orifice 88. The orientation of the first orifice 87 causes a cross-flow in the sense that the plume of the first formulation atomized at the first orifice 87 has a mean velocity with a velocity component directed transverse relative to the longitudinal axis of the actuator seat. In other words, the configuration of the first orifice 87 directs the atomized first formulation towards the atomized second formulation, so as to allow the first and second formulations to interact with each other within an actuator housing.

Cross-flow of the atomized formulations may be obtained using various different orifice configurations. For illustration, in another example the first orifice for atomizing the first formulation may have a longitudinal axis aligned with the longitudinal axis of the actuator seat, while the longitudinal axis of the second orifice for atomizing the second formulation is disposed at an angle relative to the longitudinal axis of the actuator seat. In another example, both the longitudinal axis of the first orifice for atomizing the first formulation and the longitudinal axis of the second orifice for atomizing the second formulation may be disposed at an angle relative to the longitudinal axis of the actuator seat.

A plurality of nozzle orifices may further also be formed such that the longitudinal axes of the nozzle orifices are disposed at an angle of approximately 90° relative to the longitudinal axis of the actuator seat.

Fig. 13 shows a cross sectional view of an actuator block 89 having an orifice design in which the longitudinal axes of the orifices are disposed at an angle of approximately 90° relative to the longitudinal axis of the actuator seat. The actuator block 89 defines two nozzle orifices 84 and 85. A first orifice 84 may be in fluid communication with the portion of the actuator seat in which the outer valve stem 78 is received. A second orifice 85 may be in fluid communication with the central portion of the actuator seat in which the inner valve stem 79 is received. The orifices 84 and 85 may respectively have a cylindrical shape. One or both of the orifices 84 and 85 may also be provided with a shape other than cylindrical. The first orifice 84 and second orifice 85 are spaced from each other. The longitudinal axes of the orifices 84 and 85 are parallel. The longitudinal axes of the orifices 84 and 85 are disposed at approximately 90° relative to the longitudinal axis of the actuator seats.

When the outer and inner valve stems are received in the actuator seats, the first orifice 84 is in fluid communication with the hollow space through which a first formulation is supplied and which is enclosed by the inner face of the outer valve stem 78 and the outer face of the inner valve stem 79. The second orifice 85 is in fluid communication with the hollow interior of the inner valve stem 79 through which a second formulation is supplied. Upon actuation, the first formulation moves through the outer valve stem 78 and atomizes at the first orifice 84. The second formulation moves through the inner valve stem 79 and atomizes at the second orifice 85.

Actuators having nozzle blocks defining one orifice for atomizing plural different formulations, as illustrated in Fig. 10, or plural orifices for atomizing plural different formulations, as illustrated in Figs. 11-13, may be utilized in actuators of MDIs according to various examples. The particle size distribution of a formulation may be influenced by mixing with another formulation, either pre- or post-atomization. The wide variety of possibly nozzle orifice designs provides additional versatility in adjusting the MDI so as to attain a desired particle size distribution or fine particle dose.

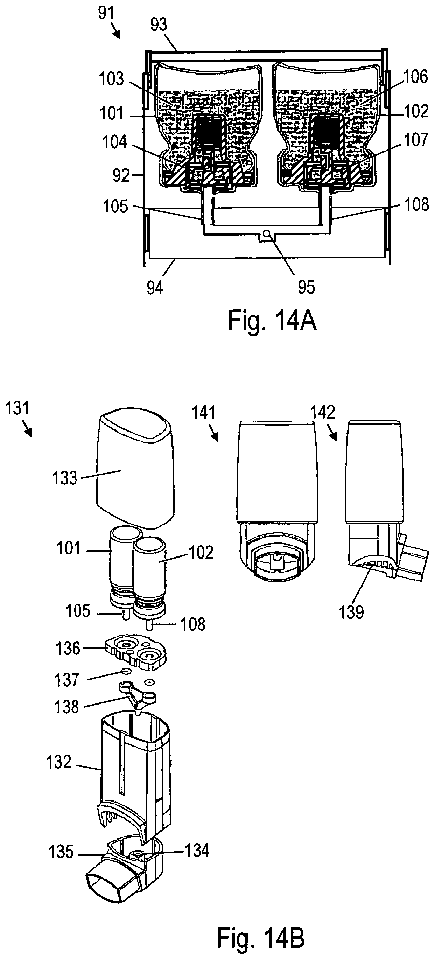

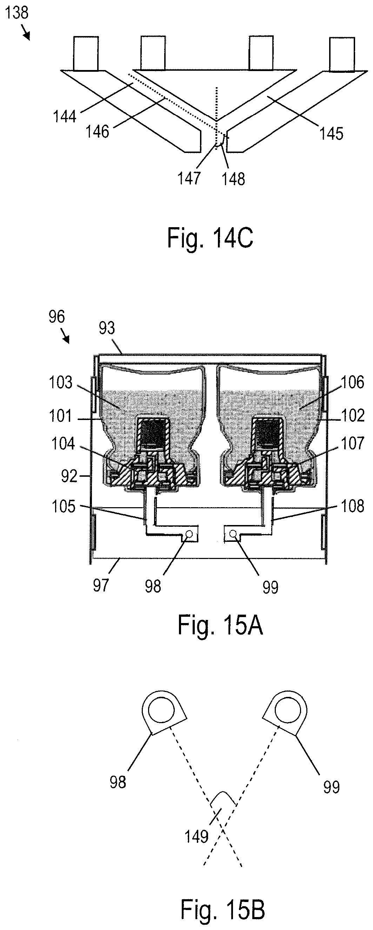

In examples described with reference to Figs. 1-13, dual-compartment vessels may be used which define both the first reservoir containing the first formulation and the second reservoir containing the second formulation. Alternative or additionally, a plurality of separate vessels may be used, as will be explained in more detail with reference to Figs. 14-16.

Fig. 14A is a schematic cross-sectional view of an MDI 91 according to an embodiment.

The MDI 91 includes an actuator 92 and at least a first vessel 101 and a second vessel 102. The first vessel 101 and the second vessel 102 are at least partially received within an actuator housing. The actuator 92, the first vessel 101 and the second vessel 102 may be configured such that the first and second vessels 101, 102 are not removeable from the actuator 92 and/or cannot be re-inserted into the actuator 92 after removal from the actuator.

The actuator 92 includes an arrangement 93 for facilitating simultaneous actuation of a first metering valve 104 associated with the first vessel 101 and of a second metering valve 107 associated with the second vessel 102. The arrangement 93 may be designed according to any one of a variety of configurations. For illustration, if the valves 104, 107 are actuated by depressing the first and second vessels 101, 102 further into the actuator 92 so as to effect a relative displacement between the vessels and their associated valve stems, the arrangement 93 may be configured as a member, e.g. an actuation plate, which distributes pressure across the vessels 101 and 102, so as to ensure that the first vessel 101 and the second vessel 102 will be jointly depressed. Suitable guiding means may be provided for the plate, to ensure that the plate travels along a pre-defined path and with a pre-defined orientation upon actuation of the MDI 91.

The actuator 92 further includes an actuator block 94 formed in an actuator housing. The actuator block 94 defines a first actuator seat for a first valve stem 105 associated with the first vessel 101 and a second actuator seat for a second valve stem 108 associated with the second vessel 102. One nozzle orifice 95 is formed in the actuator block 94. The nozzle orifice 95 is in fluid communication with both the first actuator seat for the first valve stem 105 and the second actuator seat for the second valve stem 108. A channel may lead from the first seat to the nozzle orifice 95, and a further channel may lead from the second seat to the nozzle orifice 95.

The first vessel 101 contains a first formulation 103. The second vessel 102 contains a second formulation 106. The first and second vessels may respectively be pressurized. At least one of the first and the second formulations 103, 106 may contain an active pharmaceutical agent. In embodiments, both the first and the second formulations respectively contain an active ingredient. In further embodiments, at least one of the first and second formulations does not contain an active ingredient. The first and second formulations may be different from each other. The formulation(s) which contain(s) at least one active ingredient may be an aerosol suspension formulation or an aerosol solution formulation. The formulation(s) may include the at least one active ingredient in a propellant/solvent system, and may optionally contain further excipients.

The first vessel 101 is provided with the first metering valve 104. The first metering valve 104 is configured to meter a pre-determined and consistent first dose of the first formulation 103 upon actuation. The second vessel 102 is provided with the second metering valve 107. The second metering valve 107 is configured to meter a predetermined and consistent second dose of the second formulation 106 upon actuation. The first metering valve 104 and the second metering valve 107 are distinct and operate independently. Conventional valve designs may be used. For illustration, valve designs similar to the ones described for the first and second metering valves in Figs. 3-9 may be used for the first and second valves 104 and 107, respectively. The first dose of the first formulation and the second dose of the second formulation may be equal or different in size.

Upon actuation of the MDI 91, the arrangement 93 allows the first vessel 101 and the second vessel 102 to be depressed jointly. A first metered dose of the first formulation and a second metered dose of the second formulation are delivered upon actuation. The first formulation moves through the valve stem 105 associated with the first vessel 101 and through the passageway defined in the actuator block 94 to the orifice 95. The second formulation moves through the valve stem 108 associated with the second vessel 102 and through the passageway defined in the actuator block 94 to the orifice 95. Mixing of the first and second formulations occurs prior to atomization as the expanding formulations meet at the orifice 95.

While the actuator block 94 of the actuator 92 has an orifice 95 which is disposed such that the longitudinal axis of the orifice 95 is arranged at an angle of approximate 90° relative to the longutidunal axes of the actuator block seats in which the valve stems are received, in another embodiment the actuator block 94 may be configured such that the longitudinal axis of the one orifice 95 formed in the actuator block for atomizing the first and second formulations is arranged to be essentially parallel to the the longutidunal axes of the actuator block seats in which the valve stems are received.

Fig. 14B shows an exploded view of an MDI 131 according to an embodiment. A front view 141 and side view 142 of the MDI in the assembled state are also shown in Fig. 14B. Elements or features which correspond, with regard to their configuration and/or function, to elements or features of the MDI 91 of Fig. 14A are designated by the same reference numerals.

The MDI 131 includes an actuator and at least a first vessel 101 and a second vessel 102. The first vessel 101 and the second vessel 102 are at least partially received within an actuator housing.

The actuator of the MDI 131 includes an actuator body 132, a cover 133 and a mouthpiece 135. An actuator block 134 is formed on the mouthpiece 135. The mouthpiece 135 may be a conventional actuator mouthpiece. The mouthpiece 135 and the actuator body 132 may be provided with mating engagement means, which couple the actuator body 132 and the mouthpiece 135 with each other in an operative state of the MDI 131. The engagement means of the actuator body 132 may be configured for engagement with a conventional actuator mouthpiece. This allows a mouthpiece to be selectively used for an MDI of an embodiment or for a conventional actuator.

In the assembled state of the MDI 131, the first vessel 101 and the second vessel 102 are at least partially received in the actuator body 132. The cover 133 is slideably supported on the actuator body 132 so as to displace the first vessel 101 and the second vessel 102 when the cover 133 is displaced relative to the actuator body 132. Movement of the cover 133 relative to the actuator body 132 may be guided by mating guide means provided on the cover 133 and the actuator body 132.

A support member 136, a guide member 138 and O-rings 137 are arranged within the actuator body 132. The support member 136 may extend across the interior of the actuator body 132. The support member 136 has through holes for allowing the valve stems 105, 108 of the vessels 101, 102 to pass through the support member 136. The through holes may extend parallel to a longitudinal axis of the actuator body 132.

The guide member 138 has receptacles for receiving ends of the valve stems 105, 108. The guide member 138 defines a passageway for a first metered dose of a first formulation contained in the first vessel 101. The guide member 138 defines another passageway for a second metered dose of a second formulation contained in the second vessel 102. When the actuator is assembled, an exit orifice of the guide member 138 is in communication with the actuator block 134.

The O-rings 137 are arranged in the receptacles of the guide member 138. The valve stems 105, 108 of the vessels 101, 102 are held in place and are prevented from leaking using the O-rings 137.

As best seen in the side view 142, the actuator body 132 has vents 139. The vents 139 may be formed in the base of the actuator body 132. In operation, a flow path for air is created through the actuator.

Upon actuation of the MDI 131, the cover 133 which fits over all vessels 101, 102 causes the vessels 101, 102 to be depressed together. A first metered dose of the first formulation and a second metered dose of the second formulation are delivered upon actuation. The first formulation moves through the valve stem 105 associated with the first vessel 101 and through the passageway defined in the guide member 138 to the exit orifice. The second formulation moves through the valve stem 108 associated with the second vessel 102 and through the passageway defined in the in the guide member 138 to the exit orifice. Mixing of the first and second formulations occurs prior to atomization as the formulations meet at the interconnection point of the pathways in the guide member 138.

Fig. 14C shows a cross-sectional view of the guide member 138. The guide member 138 may be comprised by the actuator block. In the guide member 138, a passageway 144 is formed for guiding the first formulation dispensed from the first vessel. Another passageway 145 is formed for guiding the second formulation dispensed from the second formulation. The first passageway 144 may be straight. The second passageway 145 may be straight. The first and second passageways may be arranged at an orifice angle relative to the longitudinal axis of the actuator body 132. I.e., a longitudinal axis 146 of the first passageway 144 may enclose an angle 148 with an axis 147 which is parallel to the longitudinal axes of both vessels 101, 102. The angle 148 is also referred to as orifice angle. The orifice angle 148 defines the orientation of the passageway(s) in the guide member 138 relative to the axis 147.

In the single orifice actuator, such as the actuators explained with reference to Fig. 14A, Fig. 14B and 14C, each formulation path, connecting the valve stem exit of the respective container and the exit orifice 95, is preferably linear and is arranged at an orifice angle included in an interval from 15° to 60° with respect to an axis parallel to the longitudinal axis of the containers in the interconnection point. The orifice angle 148 may be included in the range 0°-90°, in particular in the range 15°-60°, in particular in the range 20°-60°. The orifice angle may in particular be 20° or 30°.

While an actuator having an actuator block defining one nozzle orifice may be used, in further embodiments a plurality of nozzle orifices may be formed in the actuator block.

Fig. 15A is a schematic cross-sectional view of an MDI 96 according to a comparative example. Elements or features which correspond, with regard to their configuration and/or function, to elements or features of the MDI 91 of Fig. 14A are designated by the same reference numerals.

The MDI 96 includes an actuator 92 and at least a first vessel 101 and a second vessel 102. The first vessel 101 contains a first formulation and the second vessel 102 contains a second formulation.

The actuator 92 has an actuator block 97. The actuator block 97 defines a first actuator seat for a first valve stem 105 associated with the first vessel 101 and a second actuator seat for a second valve stem 108 associated with the second vessel 102. Two nozzle orifices 98 and 99 are formed in the actuator block 97. The first nozzle orifice 98 is in fluid communication with the first actuator seat for the first valve stem 105. The second nozzle orifice 99 is in fluid communication with the second actuator seat for the second valve stem 108. Channels may be formed in the actuator block 97 for connecting the actuator seats with the associated nozzle orifice.

Upon actuation of the MDI 96, the arrangement 93 allows the first vessel 101 and the second vessel 102 to be jointly depressed for simultaneous delivery of the first and second formulations. A first metered dose of the first formulation and a second metered dose of the second formulation are delivered upon actuation. The first formulation moves through the valve stem 105 associated with the first vessel 101 and through the passageway defined in the actuator block 97 to the first orifice 98. The second formulation moves through the valve stem 108 associated with the second vessel 102 and through the passageway defined in the actuator block 97 to the second orifice 99. Mixing of the first and second formulations occurs post atomization.

The first orifice 98 and the second orifice 99 may be configured to have any one of a wide variety of geometries. For illustration, as shown in Fig. 15A, the first orifice 98 and the second orifice 99 may be arranged to extend parallel with each other. The longitudinal axes of the first and second orifices 98, 99 may be disposed at an angle of approximately 90° relative to the longitudinal axis of the actuator seats in which the valve stems are received.

In further examples, the longitudinal axes of the first and second orifices 98, 99 may be disposed so as to be approximately parallel to the longitudinal axes of the actuator seats in which the valve stems are received, similar to the nozzle orifice configuration illustrated in Fig. 11.

In further examples, the first and second orifices 98, 99 may be provided such that their longitudinal axes are disposed at an angle relative to each other, similar to the nozzle orifice configuration illustrated in Fig. 12. Thereby, a cross-flow situation may be established in which the mean velocity of one of the first and second formulations after atomization has a component directed towards the atomized cloud of the other one of the first and second formulations.

Furthermore, for any one of the various orientations of the first orifice 98 and the second orifice 99, the distance between the first orifice 98 and the second orifice 99 may be selected in accordance with a desired point of interaction between the plumes of the first formulation and of the second formulations. Additionally or alternatively, the relative orientation between the first orifice 98 and the second orifice 99 may be selected in accordance with a desired point of interaction between the plumes of the first formulation and of the second formulations.

Fig. 15B illustrates a cross-sectional view taken in a plane in which the longitudinal axes of the first orifice 98 and the second orifice 99 are located. An impingement angle 149 may be defined to be the angle between a logitudinal axis of the first orifice 98 and a longitudinal axis of the second orifice 99.

The impingement angle may be selected from the range 0°-180°, in particular 10°-110°, in particular 15°-60°. For illustration, the impingement angle may be 15°, 60° or 90°.

By virtue of separate orifices, and the various parameteres including an orifice distance and orientation which are available to influence the plume interaction after atomization, post-atomization mixing may be used to modulate the fine particle dose, fine particle fraction, and particle size distribution of at least one of the formulations. The orifice separation distance may be defined to be the distance between a center of an exit opening of the first nozzle orifice and a center of an exit opening of the second nozzle orifice.

While MDIs having two separate vessels are shown for illustration in Figs. 14A, 14B and 15A, a greater number of vessels may be employed in further embodiments. In order to allow the vessels to be received within the actuator housing without requiring the dimensions of the actuator to be significantly increased, the separate vessels may be formed from canisters having sizes smaller than the sizes of canisters in conventional MDI systems. For illustrations, the separate vessels used may respectively have a diamter of less than 22 mm.

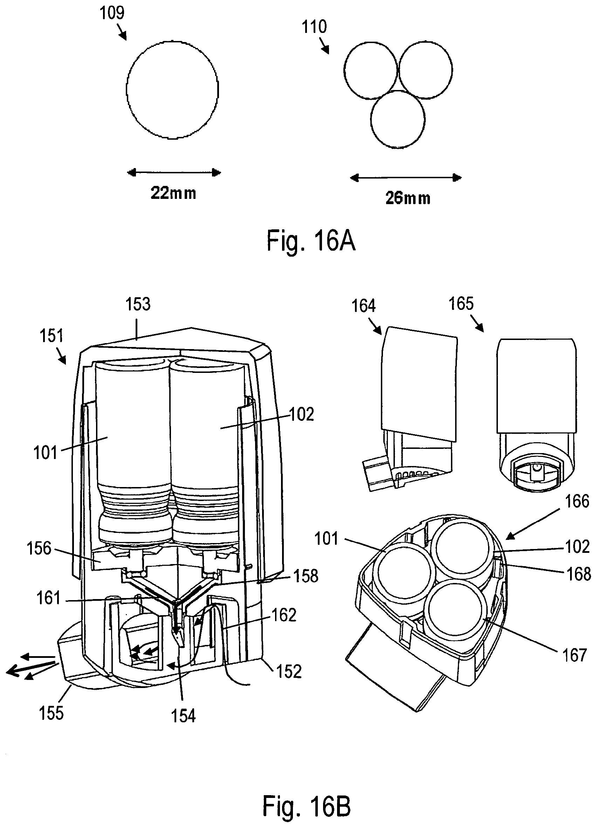

Fig. 16A illustrates a possible arrangement 110 of three canisters in plan view. Each one of the three canisters has a diameter of 13 mm. For comparison, a conventional canister having a diameter of 22 mm is shown at 109 in plan view. The three 13 mm canisters occupy a cross-sectional area which is only slightly larger than the cross-sectional area occupied by a single 22 mm canister.

Fig. 16B shows an MDI 151 according to an embodiment. The MDI 151 utilizes three canisters. Fig. 16B shows the MDI in a partially broken-away perspective view. Fig. 16B also shows a side view 164, a front view 165 and a top view 166 of the MDI 151.

The MDI 151 is generally similar in construction and operation to the MDI 131 of Fig. 14B. Elements or features which correspond, with regard to their configuration and/or function, to elements or features of the MDI 91 of Fig. 14A or to elements or features of the MDI 131 of Fig. 14B are designated by the same reference numerals.

The MDI 151 includes an actuator and at least a first vessel 101, a second vessel 102 and a third vessel 167. The first vessel 101, the second vessel 102 and the third vessel 167 are at least partially received within an actuator housing. Each one of the vessels may have a dedicated metering valve system.

The actuator of the MDI 151 includes an actuator body 152, a cover 153 and a mouthpiece 155. An actuator block 154 is formed on the mouthpiece 155. The mouthpiece 155 and the actuator body 152 may be provided with mating engagement means, which couple the actuator body 152 and the mouthpiece 155 with each other in an operative state of the MDI 151.

In the assembled state of the MDI 151, the first vessel 101, the second vessel 102 and the third vessel 167 are at least partially received in the actuator body 152. The cover 153 is slideably supported on the actuator body 152 so as to displace the first vessel 101, the second vessel 102 and the third vessel 167 when the cover 153 is displaced relative to the actuator body 152. The cover 153 covers all vessels and causes the vessels to be depressed together. Movement of the cover 153 relative to the actuator body 152 may be guided by mating guide means provided on the cover 153 and the actuator body 152.

A support member 156, a guide member 158 and O-rings are arranged within the actuator body 152. These members may have a configuration and configuration which corresponds to the one of the MDI 131 of Fig. 14B. The guide member 158 may have receptacles for receiving ends of the valve stems of the three vessels. The guide member 158 defines a first passageway for a first metered dose of a first formulation contained in the first vessel 101. The guide member 158 defines a second passageway for a second metered dose of a second formulation contained in the second vessel 102. The guide member 158 defines a third passageway for a third metered dose of a third formulation contained in the third vessel 167. When the actuator is assembled, an exit orifice of the guide member 158 is in communication with the actuator block 154.

The first, second and third passageways may be straight. The first, second and third passageways may respectively be arranged such that their longitudinal axis is arranged at an orifice angle included in an interval from 0° to 90°, in particular from 15° to 60°, in particular from 20° to 60° with respect to an axis parallel to the longitudinal axis of the containers in the interconnection point. The orifice angle may in particular be 20° or 30°.

The valve stems of the vessels 101, 102, 167 are held in place and are prevented from leaking using the O-rings.

At 166, a top view of the MDI 151 is shown with the cover 153 removed. The vessels 101, 102, 167 are arranged in a side-by-side relationship. Support ribs 168 may be formed on the actuator body 152 to support the vessels 101, 102, 167.

Upon actuation of the MDI 151, the cover 153 which fits over all vessels 101, 102, 167 causes the vessels 101, 102, 167 to be depressed together. The formulations dispensed from the vessels flow through the passageways formed in the guide member 158, as indicated at 161. Vents formed in the base of the actuator body 152 allow a flow of air 152 to be established in the actuator. The air enters the actuator at the base of the actuator.

According to embodiments, MDIs are provided which have a first reservoir of a first formulation and a second reservoir of a second formulation. When the at least one vessel is received by an actuator, the MDI allows a first metered dose of the first formulation and a second metered dose of the second formulation to be delivered. The two formulations may be metered by distinct valve systems to ensure that consistent first and second doses are delivered.

In the MDIs of the embodiments, the first dose of the first formulation and the second dose of the second formulation may both be atomized through the same orifice, or the first formulation may be atomized through a first orifice and the second formulation may be atomized through a second orifice separate from the first orifice.

Mixing of the first and second formulations may occur prior to or post atomization.

Various effects may be attained with an MDI according to embodiments.

When two formulations are delivered, the particle size distribution of each one of the formulations may be affected by the presence of the other.

When a formulation containing an active ingredient in solution or in suspension in a propellant is delivered together with another formulation containing a low volatility component, such as a glycol and in particular glycerol, the particle size distribution of the formulation containing the active ingredient may be altered by the presence of a second formulation (placebo formulation) containing the low volatility component (such as glycerol). The efficiency of an aerosol formulation in term of particle size distribution can be improved by its co-atomisation in presence of a second formulation, e.g., a placebo formulation.

When two formulations are delivered, the particle size distribution may be influenced by the volumes of the two formulations which are delivered. By using different combinations of first and second metered volumes, the mass median aerodynamic diameter may be specifically altered.

Incompatible formulations, such as formulations exhibiting physical or chemical incompatibility, may be mixed at one nozzle orifice. The incompatible formulations may be stored in independent reservoirs up until the time of delivery. This may be particularly advantageous when the simultaneous administration of a combination of active ingredients is desired or required, with the active ingredients, the solvents, propellants, or other excipients being incompatible from a chemical or physical (from a suspension and a solution formulation) point of view.

The ability to influence the particle size distribution and efficiency of a formulation by mixing it with a second formulation prior to or post atomization provides additional flexibility with regard to the formulations which can be administered using an MDI. For illustration, the first or second formulation may be selected with a view to a desired solubility, stability or drug loading capability. The resultant particle size distribution and/or fine particle dose of the atomized cloud delivered by the MDI may be matched to a desired particle size distribution and/or find particle dose by mixing with the other formulation. The consistency between the particle size distributions of two mixed formulations may be controlled by appropriately selecting their mixing process.

According to embodiments, at least one of the first formulation and the second formulation may be selected such that a particle size distribution, after atomization, of at least the other one of the first formulation and the second formulation is modulated by mixing the first formulation and the second formulation.

According to embodiments, at least one of the first formulation and the second formulation may be selected such that a fine particle dose of at least the other one of the first formulation and the second formulation is modulated by mixing the first formulation and the second formulation. The fine particle doese of the other one of the first formulation and the second formulation, which is affected by the mixing, is determined with respect to a threshold diamter which may be selected for the respective application.

MDIs of embodiments may include three reservoirs. A first reservoir may contain a first formulation, a second reservoir may contain a second formulation, and a third reservoir may contain a third formulation. The three reservoirs may be formed in separate canisters, or at least two of the reservoirs may be formed as compartments of one vessel. Upon actuation of the assembled MDI, metered doses of the first, second and third formulations may be administered. Such MDIs may be used for "triple therapies" in which three active pharmaceutical agents are delivered. An embodiment of an MDI having three separate canisters which define three reservoirs has been explained with reference to Fig. 16B.

For further illustration, the following examples are provided.

### EXAMPLES

The following examples are provided to further illustrate the effect of mixing of two formulations before atomization (Examples 1-6, 11-13, 15, 17, 18), e.g. at one nozzle orifice, or by letting two formulations interact with each other after atomization (Examples 7-10, 14, 15, 16).

Data has been obtained for a metered-dose inhaler in which the first formulation is contained in a first canister and the second formulation is contained in a second canister. The data obtained when the first and second formulation are mixed is also referred to as "dual can" or "dual-MDI" configuration in the description of the examples. Comparative data has been obtained for the delivery of one formulation from one canister. This data is also referred to as "single can" or "standard MDI" configuration in the description of examples.

### ACTUATORS

Data will be presented which have been obtained from three different actuators which allow two formulations from separate reservoirs to be simultaneously delivered.

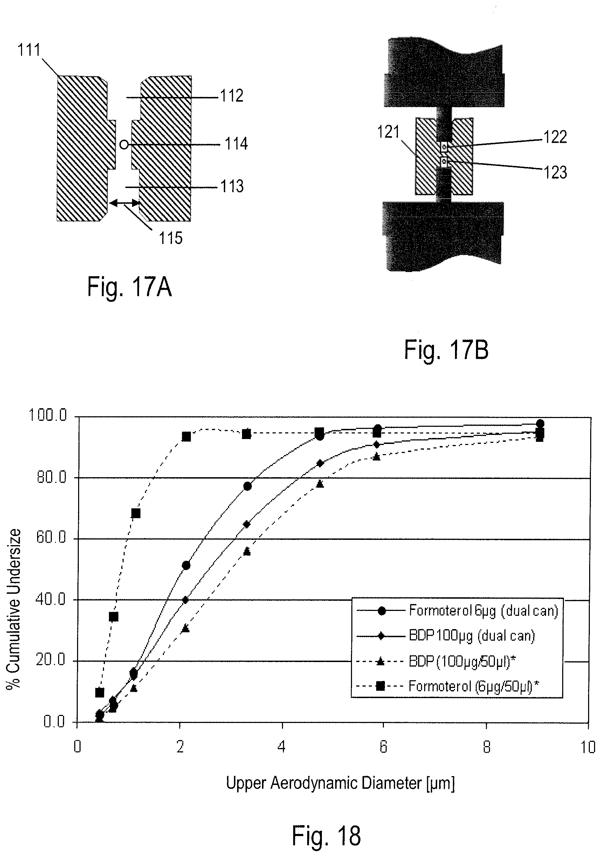

The data obtained for the "dual-MDI" or "dual can" configuration in Examples 1-6 has been obtained using two different types of actuators. A schematic cross-sectional view through the actuator block 111 of the actuators is shown in Fig. 17A. The actuator block defines two actuator seats 112 and 113 for receiving valve stems of first and second canisters, respectively. One orifice 114 is formed to extend in a direction orthogonal to the longitudinal axes of the actuator seats 112 and 113. The actuator block 111 has been arranged in an actuator housing identical to the actuator housing of the actuator used to perform the comparative measurements in the "single can" or "standard MDI" configuration. In one of the actuators (referred to as "0.22 mm actuator orifice") for the dual-MDI, the nozzle orifice 114 had a diameter of 0.22 mm. In another one of the actuators (referred to as "0.30 mm actuator orifice") for the dual-MDI, the nozzle orifice 114 had a diameter of 0.30 mm. The lengths of the orifices were respectively identical to the orifice lengths in the conventional actuator used for the comparative measurements in the single reservoir configuration.

The data obtained for the "dual-MDI" or "dual can" configuration in Examples 7-10 has been obtained using one type of actuator. A schematic cross-sectional view through the actuator block 121 of the actuator is shown in Fig. 17B. The actuator block defines two actuator seats for receiving valve stems of first and second canisters, respectively. Two orifices 122 and 123 are formed to extend in a direction orthogonal to the longitudinal axes of the actuator seats. The actuator block 111 has been arranged in an actuator housing identical to the actuator housing of the actuator used to perform the comparative measurements in the "single can" or "standard MDI" configuration. In the actuator used for the "dual MDI" measurement, the nozzle orifices 122 and 123 respectively had a diameter of 0.22 mm.

Additionally, a test rig was developed to facilitate the evaluation of the drug delivery performance of various dual reservoir-orifice configurations providing superior control, compared to the original prototype (Figs. 17A and 17B), of a range of variables for testing. When the test rig is used, the actuation of the cans is controlled remotely and both cans are situated in an inverted position (as oppose to prototypes of Figs. 17A and 17B in which one can was inverted and one was upright). The test rig developed allows accurate control of timing of actuation (either simultaneously or with delay between reservoirs) and positioning of the two or three cans relative to the expansion chambers/orifices. Configurations to examine the effects of single and dual orifice delivery are enabled, with micrometer control of separation distance between two spray orifices and various impingement angles available. The single orifice configuration utilises an orifice angle of 30°. The dual orifice configuration allows multiple separation distance and impingement angles to be investigated. The data in Examples 11-18 were obtained using the test rig.

The data obtained for the "dual" reservoir configuration in Examples 11-13 has been obtained using the test rig. The nozzle orifice had a diameter of 0.30mm. The passageways were linear. I.e., the formulation path connecting the valve stem exit of each vessel and the exit orifice was linear (cf. Fig. 14B or 14C). The passageway was aligned at an angle (orifice angle) of 20° with respect to the axis parallel to the longitudinal axes of the containers.

The data obtained for the "dual" reservoir configuration in Example 14a has been obtained using the test rig with different orifice separation distances and different orifice orientations. Two orifices were used. In Example 14b, the test rig was adapted to use, in a dual orifice configuration, an orifice diameter of 0.25mm, a separation distance of 6 mm, and an impingement angle of 60°.

The data obtained for the "dual" reservoir configuration with single orifice in Example 15 has been obtained using the test rig with the single orifice configuration (0.30 mm orifice diameter, 30° orifice angle). The data obtained for the "dual" reservoir configuration with dual orifice in Example 15 has been obtained using the the test rig with the dual orifice configuration (0.25 mm orifice diameter, 60° impingement angle, 6.0 mm separation distance).

The data obtained for the "dual" reservoir configuration with dual orifice in Example 16 has been obtained using the test rig with the dual orifice configuration (0.30 mm and 0.25 mm orifice diameter, 15° impingement angle, 6.0 mm or 10 mm separation distance between orifices).

The data obtained for the "dual" reservoir configuration with single orifice in Example 17 has been obtained using the test rig with the dual reservoir single orifice configuration with 30° orifice angle with a nozzle orifice diameter of 0.30mm.

The data obtained for the "dual" reservoir configuration with single orifice in Example 18 has been obtained using the test rig with the dual reservoir single orifice configuration with 30° orifice angle with a nozzle orifice diameter of 0.30mm.

The data in Example 19 has been obtained using the system of Fig. 14B, which has two valve-can assemblies and and an actuator with one nozzle orifice.

The data in Example 20 has been obtained using the system of Fig. 16B, which has three valve-can assemblies and an actuator with one nozzle orifice.

Comparative data for the "standard MDI" or "single can" configurations in Examples 1-6 has been obtained using standard actuators having an orifice diameter of the nozzle orifice of 0.22 mm or 0.30 mm, respectively, and orifice lengths identical to the ones of the actuators for the dual-MDI measurements.

Comparative data for the "standard MDI" or "single can" configurations in Examples 7-10 has been obtained by inserting only one can into the actuator used for the dual-can measurements.

Comparative data for the "single" reservoir configuration in Examples 11-13 has been obtained using a conventional actuator for a single reservoir-single orifice system, the orifice diameter of the nozzle orifice being 0.30mm.

Comparative data for the conventional MDI configuration having a single reservoir in Example 14 has been obtained using a conventional actuator having an orifice diameter of the nozzle orifice of 0.22mm.

Comparative data for the conventional MDI configuration having a single reservoir in Example 15 has been obtained using a conventional actuator having an orifice diameter of the nozzle orifice of 0.22mm.

Comparative data for Examples 16 and 17 were obtained using a conventional actuator having one nozzle orifice with a diameter of 0.30mm (for the BDP formulation) and a conventional actuator having one nozzle orifice with a diameter of 0.50mm (for the Salbutamol Sulphate formulation).

Comparative data for Example 18 were obtained using a conventional actuator having one nozzle orifice with diameter 0.30mm.

### METHOD

The actuator design used in some of the Examples, such as Examples 1-10, (Fig. 17A and 17B) requires one can to be in the inverted position (conventional valve) and one can to be in the upright position. Therefore, a modified metering valve provided with a dip tube was used for the can in the upright position. For the actuator design of Fig. 14B (used, for example, in Example 11), no such modification is required and conventional valves without dip tube are used.

Measurements were performed to quantify the drug delivery characteristics of MDIs delivering first and second formulations from independent reservoirs. Data has been acquired for various different first and second formulations.

Spray characteristics of the system were evaluated by an Andersen cascade impactor fitted with a USP throat (App. 1, USP 33). The particle size distribution of the sprays produced by the standard pMDI and by the pMDI according to embodiments of the invention were evaluated by Andersen Cascade Impactor (ACI) fitted with a USP throat (Chapter <601>; Apparatus 1; USP 33). The apparatus was used at a flow rate of 28.3 L/min. The multistage impactor was set up in accordance with the manufacturer's instructions. The canisters were discharged twice to waste using a standard actuator to prime the metering valve before analysis. The canisters were then fitted to the pMDI actuator system and fired once to waste. The pMDI actuator system was then attached to the USP throat using a mouthpiece adaptor. With the flow rate at 28.3 L/min, one shot was fired to the ACI and left for a period of 60 secs. This was repeated twice. Pre- and post-shot weights were recorded. After the final dose had been discharged, the actuator system, mouthpiece, USP throat and each stage from the cascade impactor were rinsed in 85:15 methanol:water. The solutions were analysed by using a UPLC/MS (Ultra Performance Liquid Chromatography / Mass Spectrometry) system for the determination of the drug deposition.

Through an ACI, the following parameters of the particles emitted by a pressurized MDI may be determined:
i) mass median aerodynamic diameter (MMAD) is the diameter around which the mass aerodynamic diameters of the emitted particles are distributed equally;
ii) delivered dose is calculated from the cumulative deposition in the ACI, divided by the number of actuations per experiment;
iii) respirable dose (fine particle dose = FPD) is obtained from the deposition from Stages 3 (S3) to filter (AF) of the ACI, corresponding to particles of diameter ≤ 5 µm, divided by the number of actuations per experiment;
iv) respirable fraction (also referred to as fine particle fraction, FPF) which is the percent ratio between the respirable dose and the delivered dose.

For Example 8, plume duration was measured using an in-line PC microphone in conjunction with Audacity 1.2.6 software.

### Example 1:

Formulation 1 and formulation 2 were mixed and fired through a single actuator orifice having a diameter of 0.22 mm or 0.30 mm. The formulations were:
Formulation 1 (Beclometasone dipropionate (BDP) formulation with a low volatility component, 25µl dose per puff):
   BDP 50µg/25µl, 13%w/w ethanol, 1.3%w/w glycerol, HFA 134a to 100%
Formulation 2 (Beclometasone dipropionate formulation with a low volatility component, 25µl dose per puff):
   Same as formulation 1.

Comparative data ("Standard MDI") were obtained for a standard actuator and a single can containing: BDP 100µg/50µl, 13% Ethanol, 1.3% Glycerol, HFA 134a to 100%.

Table 1 presents the delivered dose, the fine particle dose (FPD) ≤ 5 µm, the fine particle fraction (FPF), the mass median aerodynamic diameter (MMAD) and the number of repeats (n) performed for the respective experiment.

**Table 1: Delivery from a dual MDI for a single formulation delivered from two chambers through a single orifice in comparison with conventional MDI Delivery**

| Mean | 0.22mm actuator orifice | | 0.30mm actuator orifice | |
|---|---|---|---|---|
| | Dual MDI | Standard MDI | Dual MDI | Standard MDI |
| Delivered Dose (µg) | 99.3 | 89.8 | 95.6 | 89.1 |
| FPD ≤ 5 µm (µg) | 44.6 | 41.9 | 27.1 | 26.2 |
| FPF (%) | 45.0 | 46.6 | 28.3 | 29.4 |
| MMAD (µm) | 2.9 | 3.0 | 3.0 | 3.3 |
| n | 3 | 3 | 3 | 3 |

The performance and particle size distributions obtained for a Dual MDI delivering doses from the independent cans are similar to that of a conventional MDI delivering 100µg/50ul (i.e. the sum of formulations 1 and 2).

This example shows that if formulations 1 and 2 are the same then drug delivery characteristics obtained with a dual MDI are similar to that of a standard MDI.

### Example 2:

Formulations 3 and 4 were mixed and fired through a single 0.22mm actuator orifice. The formulations were:
Formulation 3 (formoterol formulation, 25µl dose per puff):
   Formoterol fumarate 6µg/25µl, 12%w/w ethanol, 0.0474%w/w HCI (1M), HFA 134a to 100%
Formulation 4 (BDP formulation with a low volatility component, 25µl dose per puff): BDP 100µg/25µl, 12%w/w ethanol, 1.3%w/w glycerol, HFA 134a to 100%

Comparative data were obtained by firing a dose of BDP formulation or formoterol formulation through a standard actuator having an orifice diameter of 0.22 mm. Formulations in the single can configuration: BDP 100µg/50µl, 13% Ethanol, 1.3% Glycerol, HFA 134a to 100%; or: Formoterol fumarate 6µg/50µl, 12% EtOH, 0.024% HCI (1M), HFA 134a to 100%.

The drug delivery characteristics from the dual MDI system are presented in Table 2 and Fig. 18. The particle size distribution of each of the formulations is affected by the presence of the other. Data obtained for the dual MDI system are shown as circles and diamonds, respectively, in Fig. 18. Comparative data are indicated by squares and triangles, respectively.

This example shows that the particle size distribution of one formulation can be altered by the presence of a second formulation.

**Table 2: Dual-MDI Delivery for two formulations with different particle size distributions**

| Mean | BDP Dual MDI | BDP Standard MDI | Formoterol Dual MDI | Formoterol Standard MDI |
|---|---|---|---|---|
| Delivered Dose (µg) | 97.9 | 99.3 | 3.2 | 4.1 |
| FPD ≤ 5 µm (µg) | 52.5 | 44.6 | 1.9 | 2.1 |
| FPF (%) | 53.6 | 45.0 | 61.1 | 51.9 |
| MMAD (µm) | 2.5 | 2.9 | 2.1 | 0.9 |
| n | 2 | 3 | 2 | 3 |

### Example 3:

Formulations 3 and 5 were mixed and fired through a single 0.22 mm actuator orifice. These formulations were:
Formulation 3 (Formoterol Formulation, 25µl dose per puff):
   Formoterol fumarate 6µg/25µl, 12%w/w ethanol, 0.0474%w/w HCI (1M), HFA 134a to 100%
Formulation 5 (Placebo Formulation with a low volatility component, 25µl dose per puff):
   13%w/w ethanol, 1.3%w/w glycerol, HFA 134a to 100%

Comparative data were obtained by firing Formoterol formulation through a standard actuator having an orifice diameter of 0.22 mm. Formulation in the single can configuration: Formoterol fumarate 6µg/50µl, 12% EtOH, 0.024% HCI (1M), HFA 134a to 100%.

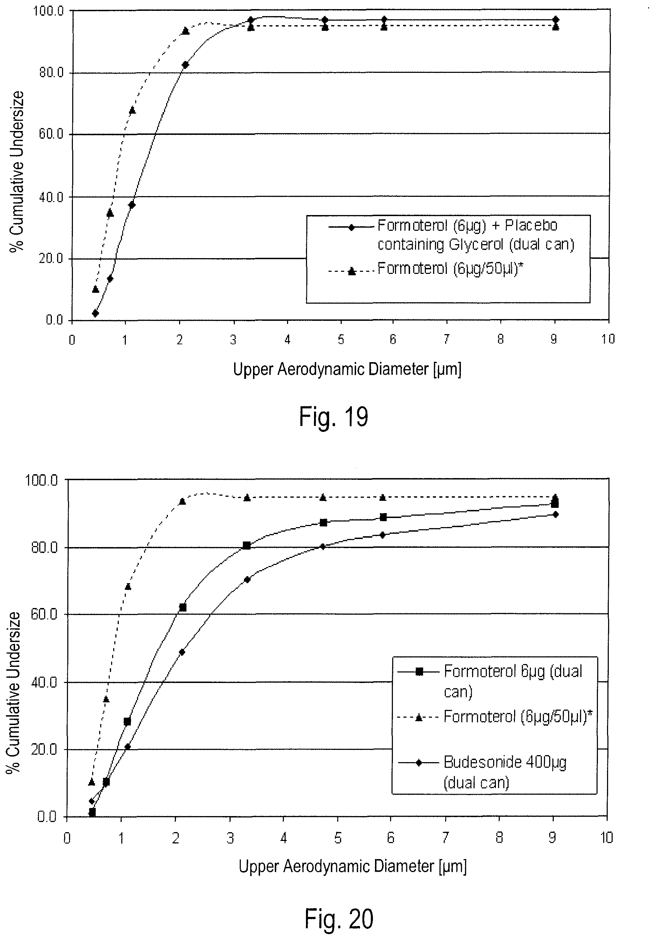

The drug delivery characteristics from the Dual-MDI system are presented in Table 3 and Fig. 19. The particle size distribution of the formoterol formulation (indicated by diamonds for the comparative data in Fig. 19) is affected by the presence of the placebo formulation. The data obtained for mixing formulations 3 and 5 are indicated by diamonds in Fig. 19.

This example demonstrates that the particle size distribution of one formulation can be altered by the presence of another placebo formulation containing glycerol as low volatility component.

**Table 3: Dual-MDI Delivery of a formoterol formulation and placebo with different particle size distributions**

| Mean | Formoterol Dual MDI | Formoterol Standard MDI |
|---|---|---|
| Delivered Dose (µg) | 4.2 | 4.1 |
| FPD ≤ 5 µm (µg) | 2.7 | 2.1 |
| FPF (%) | 64.0 | 51.9 |
| MMAD (µm) | 1.5 | 0.9 |
| n | 3 | 3 |

### Example 4:

Formulations 6 and 7 were mixed and fired through a single 0.22mm actuator orifice. The two formulations were:
Formulation 6 (high ethanol content BDP formulation, 25µl dose per puff):
   BDP 100µg/25µl, 26%w/w ethanol, 1.3%w/w Glycerol, HFA 134a to 100%
Formulation 7 (100% HFA 134a, 25µl dose per puff):
   100%w/w HFA 134a delivered through a 25µl valve

Comparative data were obtained by firing BDP formulation through a standard actuator having an orifice diameter of 0.22 mm. Formulation in the single can configuration: BDP 100µg/25µl, 26%w/w ethanol, 1.3%w/w Glycerol, HFA 134a to 100%.

The drug delivery characteristics from a standard MDI and the Dual-MDI system are presented in Table 4. The efficiency of the high ethanol (26%w/w) formulation is improved when mixed with HFA134a prior to being atomized through the nozzle. The FPD increases from 27µg to 41µg and the FPF increases from 27% to 48%, due to mixing with HFA134a prior to the nozzle.

This example demonstrates that the efficiency of a high ethanol content formulation can be improved by the presence of another placebo formulation.

**Table 4: Standard and Dual-MDI Delivery of a high ethanol (26%w/w) BDP 100µg formulation**

| Mean | BDP Standard MDI* | BDP Dual-MDI |
|---|---|---|
| Delivered Dose (µg) | 100.6 | 86.4 |
| FPD ≤ 5 µm (µg) | 26.7 | 41.1 |
| FPF (%) | 26.5 | 47.5 |
| MMAD (µm) | 3.5 | 2.7 |
| n | 2 | 2 |

### Example 5:

Formulations 8 and 3 were mixed and fired through a single 0.22mm actuator orifice. These two formulations were:
Formulation 8 (High Dose Budesonide Formulation, 63µl dose per puff):
   Budesonide 400µg/63µl, 15%w/w Ethanol, 2%w/w Water, 0.002%w/w Phosporic acid (15M), 0.2%w/w glycerol, HFA 134a to 100%
Formulation 3 (Formoterol Formulation, 25µl dose per puff):
   Formoterol fumarate 6µg/25µl, 12%w/w ethanol, 0.0474%w/w HCl (1M), HFA 134a to 100%

Formulation 8 and formulation 3 are examples for incompatible formulations. The stability of a formulation depends upon the container type and excipients within the formulation. The Dual-MDI system offers the opportunity to use different container types (or container coatings) for each formulation. In addition, formulation stabilisers or solubilisers may be necessary in one formulation but these may be incompatible with the second formulation. This example combines two incompatible formulations and shows that good drug delivery is achievable by mixing the formulations at the nozzle.

Comparative data were obtained by firing Formoterol formulation through a standard actuator having an orifice diameter of 0.22 mm. Formulation in the single can configuration: Formoterol fumarate 6µg/50µl, 12% EtOH, 0.024% HCl (1M), HFA 134a to 100%.

The drug delivery characteristics from the Dual-MDI system are presented in Table 5 and Fig. 20 (with the comparative data being indicated by triangles). The particle size distribution of the formoterol formulation is affected significantly by the presence of the budesonide formulation. The MMAD of the formoterol formulation approaches that of budesonide.

This example shows that two incompatible formulations can be mixed at the actuator nozzle orifice.

**Table 5: Dual-MDI Delivery of a formoterol formulation and high dose Budesonide formulation with different particle size distributions**

| Mean | Formoterol | Budesonide |
|---|---|---|
| Delivered Dose (µg) | 3.5 ± 0.7 | 386.8 ± 17.6 |
| FPD ≤ 5 µm (µg) | 1.9 ± 0.2 | 162.1 ± 1.7 |
| FPF (%) | 53.6 ± 6.6 | 42.0 ± 1.9 |
| MMAD (µm) | 1.7 ± 0.1 | 2.2 ± 0.2 |
| n | 3 | 3 |

### Example 6:

A BDP solution formulation and a budesonide solution formulation with a low volatility component were respectively mixed and fired through a single 0.22 mm actuator orifice. Measurements were performed for different metered volumes of the BDP formulation and for different metered volumes of the budesonide formulation. This example shows that the particle size distributions can be affected by controlling the metered volumes of the different solutions.

The BDP solution formulation was respectively selected from the following formulations 9-11, and the budesonide formulation with a low volatility compound was respectively selected from the following formulations 12-14:
Formulation 9 (BDP solution formulation, 50µg/25µL dose per puff):
   0.18% w/w BDP, 15%w/w ethanol, HFA 134a to 100%
Formulation 10 (BDP solution formulation, 50µg/50µL dose per puff):
   0.09% w/w BDP, 15% w/w ethanol, HFA 134a to 100%
Formulation 11 (BDP solution formulation, 50µg/100µL dose per puff):
   0.04% w/w BDP, 15% w/w ethanol, HFA 134a to 100%
Formulation 12 (budesonide solution formulation with a low volatility component, 50µg/25µL dose per puff):
   0.18% w/w budesonide, 15% w/w ethanol, 1.3% w/w glycerol, 0.002% w/w phosphoric acid (15M), HFA 134a to 100%
Formulation 13 (budesonide solution formulation with a low volatility component, 50µg/50µL dose per puff):
   0.09% w/w budesonide, 15% w/w ethanol, 1.3% w/w glycerol, 0.002% w/w phosphoric acid (15M), HFA 134a to 100%
Formulation 14 (budesonide solution formulation with a low volatility component, 50µg/100µL dose per puff):
   0.04% w/w budesonide, 15% w/w ethanol, 1.3% w/w glycerol, 0.002% w/w phosphoric acid (15M), HFA 134a to 100%

Comparative data were obtained by firing formulations 9-14 in a single can configuration using an actuator having a 0.22 mm nozzle orifice.

The drug delivery characteristics from the single-can configuration are presented in Table 6. The MMAD of the BDP (50µg) and budesonide (50µg) formulations are not significantly affected by metered volume when fired in the single can configuration using a 0.22 mm actuator (Table 6). Values of 1.1-1.2 µm and 3.0-3.1 µm are consistently obtained for BDP and budesonide MMAD, respectively.

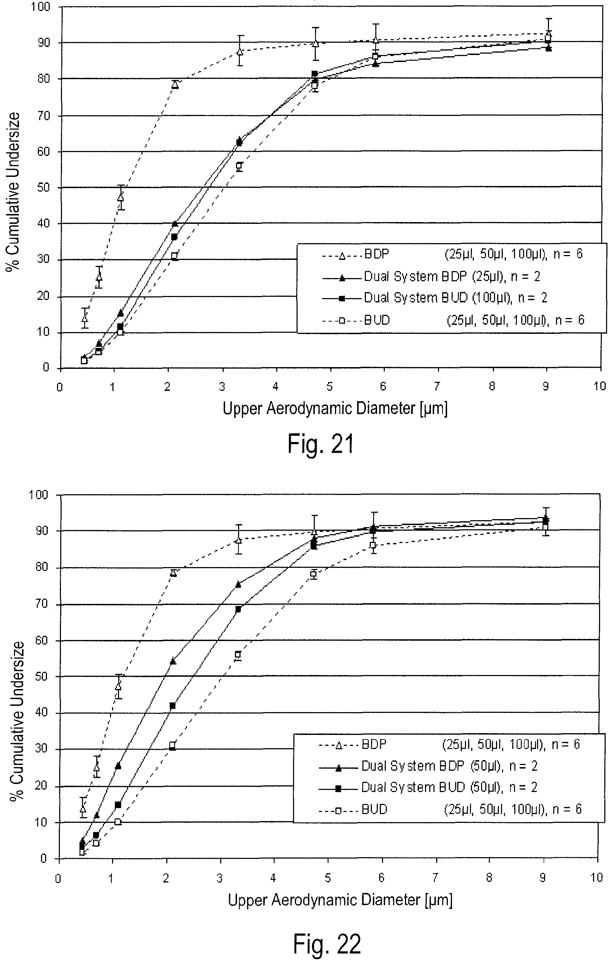

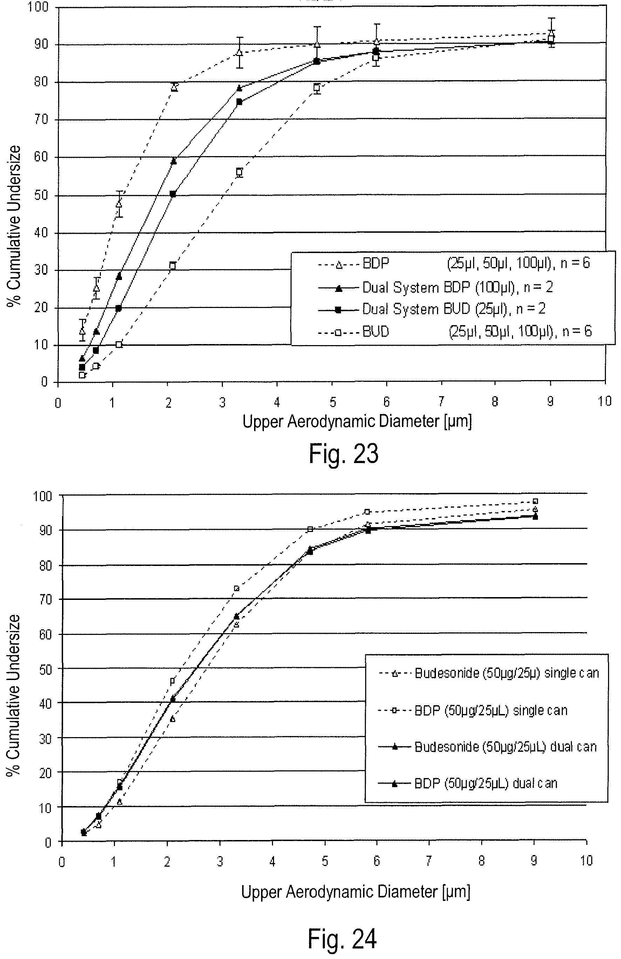

The drug delivery characteristics from the Dual-MDI system are presented in Table 7 and Figs. 21-23 (with the comparative data indicated by open symbols and the DUA-MDI data indicated by solid symbols). In contrast to the single-can configuration, when different combinations of metered volumes are used in the dual can configuration, the MMAD can be specifically altered (Table 7). Larger volumes of the budesonide formulation relative to the BDP formulation causes a shift in the MMAD of BDP (2.6 µm) towards that of budesonide (2.7µm) (Fig. 21). Equal volumes of both formulations result in a shift of the MMAD towards the centre of the two original values; values of 1.9 µm and 2.4 µm are obtained for BDP and budesonide MMAD, respectively (Fig. 22). Larger volumes of the BDP formulation relative to the budesonide formulation, including the low volatility component, causes a shift in the MMAD of budesonide (2.1µm) towards that of BDP (1.8µm) (Fig. 23). However, the shift is modulated by the presence of the low volatility component (glycerol) in the budesonide formulation.

This example shows the ability of an MDI which simultaneously delivers first and second formulations to modify the particle size distribution. In particular, this example shows that the particle size distributions of the formulations can be modulated by adjusting the metered volume. This allows particle size distributions to be modulated via the metered volumes.

**Table 6: Aerosol characteristics of BDP and budesonide (50µg dose) at different metered volumes using a 0.22 mm actuator**

| Mean | BDP 50µg | | | Budesonide 50µg | | |
|---|---|---|---|---|---|---|
| | 25µL | 50µL | 100µL | 25µL | 50µL | 100µL |
| Delivered Dose (µg) | 52.0 | 45.6 | 48.8 | 55.4 | 45.4 | 47.7 |
| FPD ≤ 5 µm (µg) | 31.1 | 24.7 | 19.2 | 27.0 | 18.2 | 18.0 |
| FPF (%) | 59.8 | 54.0 | 39.4 | 48.8 | 40.0 | 37.7 |
| MMAD (µm) | 1.2 | 1.2 | 1.1 | 3.0 | 3.1 | 3.0 |
| n | 2 | 2 | 2 | 2 | 2 | 2 |

**Table 7: Aerosol characteristics of dual can BDP and budesonide using different combinations of metered volume**

| Mean | BDP 25µL | | BDP 50µL | | BDP 100µL | |
|---|---|---|---|---|---|---|
| | BUD 100µL | | BUD 50µL | | BUD 25µL | |
| | BDP | BUD | BDP | BUD | BDP | BUD |
| Delivered Dose (µg) | 49.4 | 35.4 | 38.2 | 44.8 | 45.5 | 53.6 |
| FPD ≤ 5 µm (µg) | 18.5 | 14.5 | 16.8 | 18.0 | 18.6 | 22.0 |
| FPF (%) | 37.3 | 41.0 | 44.1 | 40.1 | 40.8 | 40.9 |
| MMAD (µm) | 2.6 | 2.7 | 1.9 | 2.4 | 1.8 | 2.1 |
| n | 2 | 2 | 2 | 2 | 2 | 2 |

### Example 7:

Formulations 15 and 16 were mixed and fired through the dual orifice actuator, as shown at 121 in Fig. 17B. The two formulations used were:
Formulation 15 (budesonide formulation with a low volatility component, 25µl dose per puff):
   0.18% w/w Budesonide (50µg/25µl), 13%w/w ethanol, 1.3%w/w glycerol, HFA 134a to 100%
Formulation 16 (BDP formulation with a low volatility component, 25µl dose per puff): 0.18% w/w BDP (50µg/25µl), 13%w/w ethanol, 1.3%w/w glycerol, HFA 134a to 100%

For the individual measurements, formulation 15 was fired in the inverted position and formulation 16 was fired in the upright position using the dual orifice actuator.

Table 8 and Fig. 24 present the drug delivery characteristics and cumulative undersize of each formulation fired either simultaneously (indicated as solid symbols in Fig. 24, "dual can") or individually (indicated as empty symbols in Fig. 24, "single can") from the dual orifice actuator. The upright orientation of the BDP formulation results in a reduced delivered dose due to retention on the stem, valve and actuator. When the formulations are actuated simultaneously the delivered dose is improved due to the interaction between the plumes reducing deposition on the actuator. The particle size distributions of the formulations actuated simultaneously are comparable to those produced by independent actuation. This shows that two similar formulations atomized simultaneously may produce the same particle size distribution as if actuated independently.

This example shows that similar particle size distributions can be obtained when simultaneously actuating two near-identical formulations, compared to individual actuation. This example also demonstrates the mixing of formulations post-atomization following simultaneous actuation from two orifices situated in close proximity.

**Table 8 Aerosol characteristics of budesonide and BDP actuated individually or simultaneously through the dual orifice actuator**

| Mean | Single can | | Dual can | |
|---|---|---|---|---|
| | Budesonide | BDP | Budesonide | BDP |
| Delivered Dose (µg) | 53.1 | 36.0 | 42.7 | 41.5 |
| FPD ≤ 5 µm (µg) | 23.7 | 17.0 | 12.4 | 12.7 |
| FPF (%) | 44.7 | 47.2 | 28.9 | 30.6 |
| MMAD (µm) | 2.7 | 2.3 | 2.5 | 2.5 |
| N | 2 | 2 | 2 | 2 |

### Example 8:

Formulation combinations 17a-18a and 17b-18b are fired through the dual orifice actuator, as shown at 121 in Fig. 17B. Plume duration was measured for the simultaneous actuation of two 25µL valves and two 63µL valves and compared with plume duration for the individual cans. This example demonstrates that metering volume can be doubled without increasing the plume duration. The two formulation combinations were:
25µL formulation combinations:
   Formulation 17a: 0.18% w/w budesonide; 13% w/w ethanol; 1.3% w/w glycerol; HFA 134a to 100%; and
   Formulation 18a: 0.18% w/w BDP; 13% ethanol; HFA 134a to 100%
63µl formulation combinations:
   Formulation 17b: 0.07% w/w budesonide; 13% w/w ethanol; 1.3% w/w glycerol; HFA 134a to 100%; and
   Formulation 18b: 0.07% w/w BDP; 13% w/w ethanol; HFA 134a to 100%; and
   The plume duration measurements are summarized in Table 9. The standard deviation (SD) and relative standard deviation (RSD) are also given in Table 9. The duration of the plume from the combined formulations is comparable to the plume durations of the individual cans.

This example demonstrates that the metered volume can be doubled without increasing the plume duration using the dual can configuration when the formulations are fired through two nozzle orifices.

**Table 9: Plume duration of individual and dual cans**

| Can | Valve size | Plume duration (s) | RSD (%) | SD (s) |
|---|---|---|---|---|
| BDP- upright | 25µL | 0.317 | 6.2 | 0.02 |
| Budesonide- inverted | | 0.246 | 2.1 | 0.005 |
| Dual can | | 0.321 | 7.4 | 0.024 |
| BDP- upright | 63µL | 0.545 | 2.8 | 0.015 |
| Budesonide- inverted | | 0.496 | 3.0 | 0.015 |
| Dual can | | 0.552 | 4.0 | 0.022 |

### Example 9:

Formulations 17a and 18a of the previous Example 8 were fired through the dual orifice actuator and the aerosol characteristics were compared with each formulation fired individually. This example demonstrates the mixing of formulations post-atomization following simultaneous actuation from two orifices situated in close proximity. The two formulations used were:
Formulation 17a (Budesonide Formulation with low volatility component, 25µl dose per puff):
   0.18% w/w Budesonide (50µg/25µl), 13%w/w ethanol, 1.3%w/w glycerol, HFA 134a to 100%
Formulation 18a (BDP Formulation, 25µl dose per puff):
   0.18% w/w BDP (50µg/25µl). 13%w/w ethanol, HFA 134a to 100%

For the individual comparative measurements, formulation 17a (budesonide) was fired in the inverted position and formulation 18a (BDP) was fired in the upright position using the dual orifice actuator.

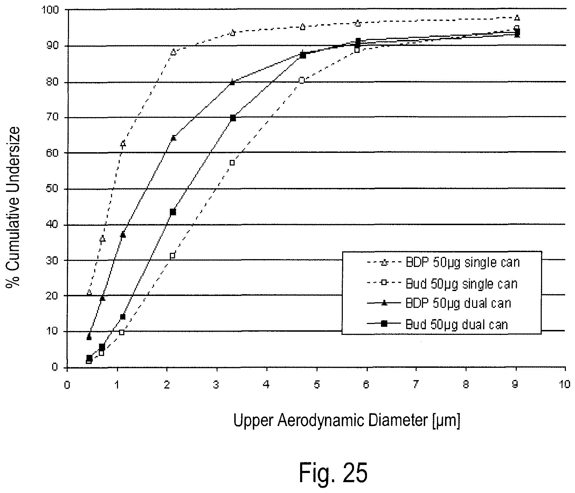

Table 10 and Fig. 25 present the aerosol characteristics of budesonide (50µg/25µL) and BDP (50µg/25µL) actuated individually (empty symbols in Fig. 25, "single can") or simultaneously (solid symbols in Fig. 25, "dual can") using the dual orifice actuator. The upright orientation of the BDP delivered from single can formulation results in a reduced delivered dose due to retention on the stem, valve and actuator. When the formulations are actuated simultaneously the delivered dose is improved due to the interaction between the plumes reducing deposition on the actuator. In addition, the MMAD of BDP is increased (from 0.9 to 1.5 µm) and budesonide is decreased (from 3.0 to 2.4 µm). This can be attributed to post-atomization mixing of the formulations, causing a change in the measured particle size distribution.

This example shows the mixing of formulations post-atomization. The example also shows that the particle size distribution can be modulated post-atomization, indicative of particle formation occurring after the orifice.

**Table 10: Aerosol characteristics of budesonide and BDP actuated individually ("single can") or simultaneously ("dual can") through the dual orifice actuator**

| Mean | Single can | | Dual can | |
|---|---|---|---|---|
| | Budesonide | BDP | Budesonide | BDP |
| Delivered Dose (µg) | 54.2 | 29.1 | 45.1 | 40.8 |
| FPD ≤ 5 µm (µg) | 25.6 | 16.2 | 13.2 | 12.7 |
| FPF (%) | 47.1 | 55.8 | 29.3 | 31.1 |
| MMAD (µm) | 3.0 | 0.9 | 2.4 | 1.5 |
| N | 2 | 2 | 2 | 2 |

### Example 10:

The plumes produced using the dual-orifice actuator were visualized using high speed imaging, after simultaneous actuation of the two valve systems.

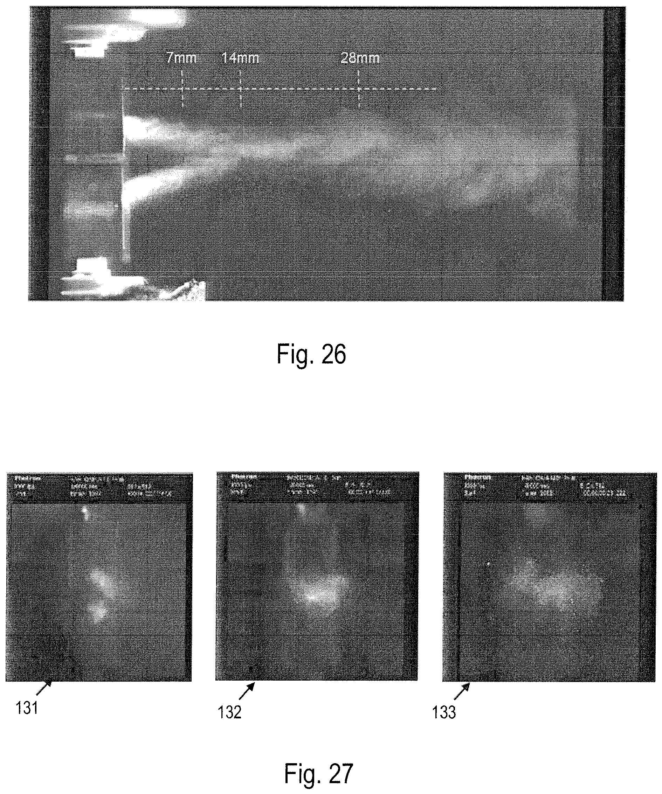

Fig. 26 shows a side view of the plumes. Fig. 27 shows cross-sections taken at the positions of 7 mm (shown at 131 in Fig. 27), 14 mm (shown at 132 in Fig. 27) and 28 mm (shown at 133 in Fig. 27) from the orifices.

The images shown in Fig. 26 and 27 demonstrate the interaction of the plumes following the simultaneous actuation of two formulations. The images captured demonstrate the combination of the two individual plumes and a change in the plume geometry after impingement, as evident upon comparison of image 131 with image 132.

The angle and distance between the nozzle orifices will determine the point of impingement and the type of mixing that occurs post-atomization.

### Example 11:

This example also demonstrates the modulation of the PSD of a first formulation by simultaneous delivery of a second formulation.

BDP Formulation 9 and Budesonide Formulation 12 (see Example 6) were delivered through a 0.30mm diameter orifice, using the actuator of Fig 14B.

For obtaining comparative data ("Single", referring to single reservpor), identical formulations packaged in 50µl valves (Formulation 10 and 13 of Example 6) were used, to match the total volume of the dual dose, and delivered through conventional MDI systems having actuators with an orifice diameter of 0.30 mm.

Experiments carried out using the actuator of Fig. 17A showed that delivery of two distinct formulations through a single orifice causes their PSDs to become more similar (see Example 6). One effect of a system according to Fig. 14B, where each formulation path, connecting the valve stem exit of each container and the exit orifice 95, is linear and is aligned at an angle of 20° with respect to the axis parallel to the longitudinal axis of the containers in the interconnection point is that it is easier to attain total synchronization of actuation of the two cans, allowing simultaneous mixing of the two formulations.

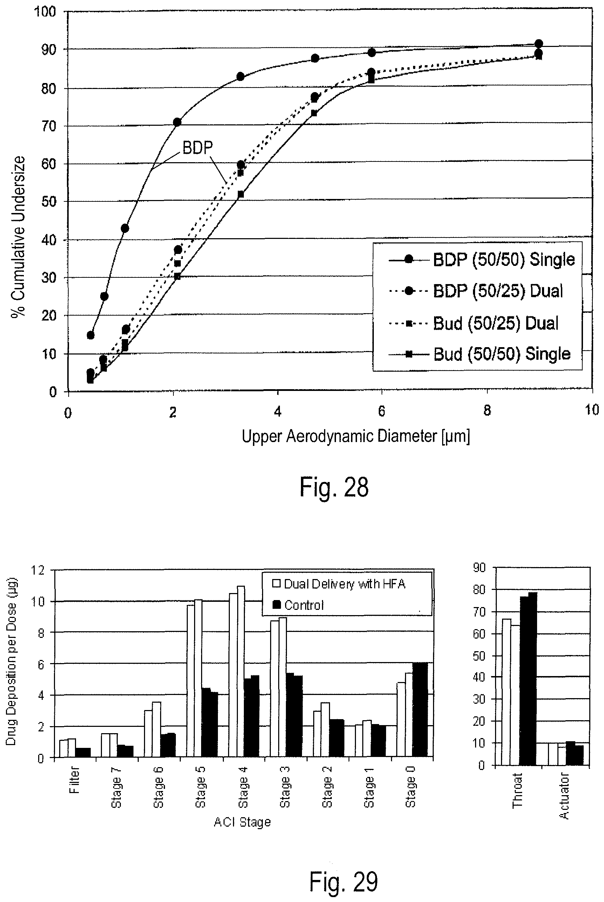

The drug delivery characteristics for the dual-can single-orifice MDI system are presented in Table 11 and Fig. 28. The data were obtained using the test rig in a configuration having a single orifice. Dual delivery results in almost identical particle size distribution for the two formulations, indicated in Fig. 28 by the data points connected by broken lines, with the MMAD of the BDP shifting from 1.3µm (data obtained for conventional MDI) to 2.8 µm, and the Budesonide from 3.2µm to 2.9µm. This demonstrates that high synchronicity of actuation is desired for simultaneous mixing.

The MMAD of the BDP Formulation 9 shows a greater change than that of the Budesonide Formulation 12. This can be attributed to the influence of glycerol (low volatility component) on the BDP particles during the mixing process, which begins pre-orifice.

In addition to the shift in MMAD, the Fine Particle Fractions (% FPF) are also comparable (see Table 11) falling between those obtained for the two single MDIs (27% and 35%).

**Table 11: Aerosol characteristics of dual can delivery of BDP and budesonide formulations for a dual-can single-orifice MDI**

| Mean | BDP | BDP | Bud | Bud |
|---|---|---|---|---|
| | (50/50) | (50/25) | (50/25) | (50/50) |
| | Single | Dual | Dual | Single |
| Metered Dose (µg) | 52.7 | 59.5 | 63.1 | 48.7 |
| Delivered Dose (µg) | 47.1 | 55.2 | 56.2 | 44.4 |
| FPD (µg) | 16.6 | 16.3 | 16.9 | 12.1 |
| FPF (%) | 35.2 | 29.5 | 30.1 | 27.2 |
| MMAD (µm) | 1.3 | 2.8 | 2.9 | 3.2 |
| n | 2 | 2 | 2 | 2 |

### Example 12:

This example demonstrates that HFA may be used to increase atomisation of high ethanol formulations.

Formulation 19 and 20 are fired through a system having two reservoirs and a single orifice, similar to the configuration illustrated in Fig. 14B. The dose volume is respectively 25 µl. This was compared with data from the delivery of the formulation 19 (high ethanol formulation) through a conventional single reservoir actuator having an orifice diameter of 0.30mm.
Formulation 19 (high ethanol formulation): BDP (100µg/25µl), 26% w/w ethanol
Formulation 20: HFA 134a

The delivery characteristics for the dual reservoir - single orifice MDI system are presented in Table 12 and Fig. 29. Fig. 29 shows the drug deposition measured with the ACI. In Fig. 29, unfilled bars indicate ACI drug deposition for the dual reservoir - single orifice actuator. Solid bars indicate the comparative data obtained with a conventional actuator having one reservoir only.

Mixing with HFA increases atomisation of the high ethanol formulation. The MMAD is reduced from 3.7µm to 2.8µm with the addition of HFA (see Table 12), indicating an increase in particle break up. The FPD also increases from 18.1µg, measured for the conventional MDI data, to 36.3µg for the dual can - single orifice MDI, and induction port deposition is decreased.

**Table 12: Aerosol characteristics of dual-can delivery of a high ethanol formulation for a dual-can single-orifice MDI**

| | Dual | | Single (Control) | |
|---|---|---|---|---|
| Metered Dose (µg) | 120.8 | 119.0 | 114.6 | 114.6 |
| Delivered Dose (µg) | 110.9 | 111.1 | 104.3 | 106.0 |
| FPD (µg) | 35.3 | 37.2 | 18.2 | 17.9 |
| FPF (%) | 31.9 | 33.5 | 17.5 | 16.9 |
| MMAD (µm) | 2.8 | 2.8 | 3.7 | 3.7 |

### Example 13:

BDP Formulation 9 and Budesonide Formulation 12 (see Example 6, but with dose volumes per puff of 25µl or 100µl) were fired simultaneously through a 0.30 mm diameter orifice using a configuration with a single orifice, as schematically illustrated for the actuator according to Fig 14B. The dose volumes were set to be different, with one of the dose volumes being 25µl and the other one of the dose volumes being 100µl.

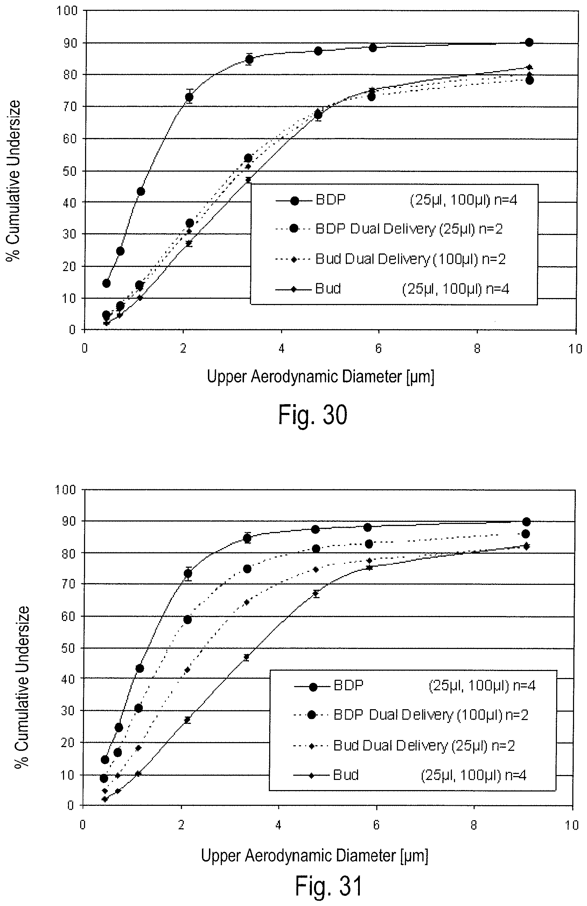

Figs. 30 and 31 shows the results from delivery of unequal volumes of BDP Formulation 9 and Budesonide Formulation 12. In both cases, the two cans were activated simultaneously, meaning that the 25µl dose duration is contained within that of the longer 100µl dose duration.

Comparative measurements (indicated as "BDP" and "Bud" in Figs. 30 and 31) were performed using a conventional single reservoir MDI, with the nozzle orifice diameter being 0.30mm.

The formulation containing glycerol (low volatility component) has a greater influence on the PSD of the BDP formulation (without low volatility component). For the case where a 25µl dose volume is used for the BDP formulation, the BDP MMAD shifts from 1.2µm (data obtained for conventional MDI) to 3.1 µm when delivered with 100µl of budesonide formulation (Figure 30). The MMAD of the budesonide formulation shifts only slightly, from 3.5µm to 3.2µm. This result is similar to that of Figure 28, where equal volumes of the two formulations were delivered simultaneously. The difference is, however, that increasing the influence of the glycerol containing formulation compared with the BDP shifts the MMADs of the two formulations further towards that of the budesonide data generated for the conventional MDI comparison (compare Figs. 30 and 31).

Equal dose volumes of the two formulations produce similar MMADs which are 2.8 and 2.9µm (see Table 11 above).

### Example 14a:

This example demonstrates that impingement angles of plumes delivered through two orifices and orifice separation distance affect atomization and plume mixing.

BDP Formulation 9 and Budesonide Formulation 12 (see Example 6) were delivered through the test rig in a configuration having two orifices. I.e., a dual reservoir - dual orifice configuration was used. This was done for various actuators having different orifice separation distances and impingement angles.

To obtain comparative data ("Single can"), identical formulations were used. The formulations were delivered through conventional actuators having an orifice with a diameter of 0.22mm. The conventional single reservoir MDIs had 50µl valves. The comparative data gave MMADs of 1.3µm for the BDP formulation, and 3.1µm for the budesonide formulation, for comparison.

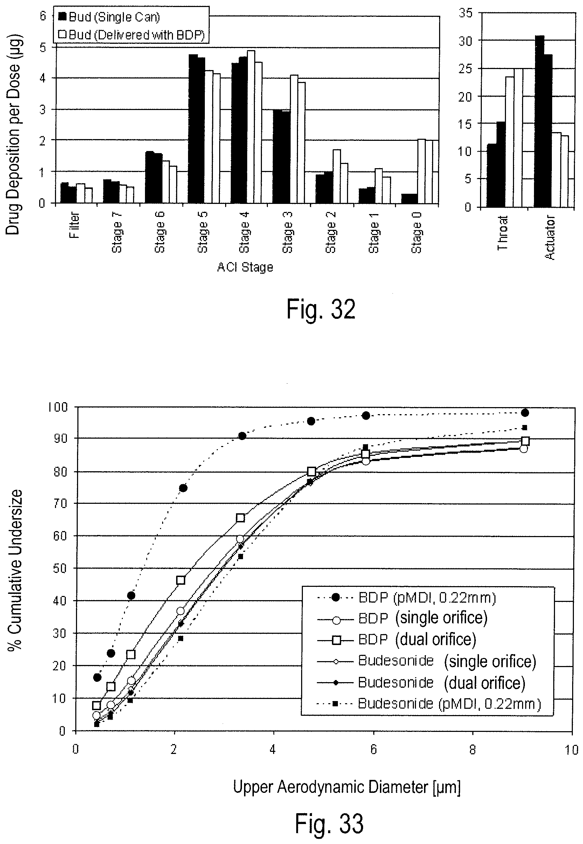

The drug delivery characteristics for a dual reservoir - dual orifice MDI system with the test rig are presented in Table 13. The orifice diameter was also allowed to vary. Table 14 and Fig. 32 show the drug delivery characteristics for the dual reservoir - dual orifice MDI system compared to the comparative data obtained for the single reservoir - single orifice MDI system.

**Table 13: Effect of Separation Distance and Impingement Angle on MMAD**

| Impingement Angle (°) | Orifice Diameter (mm) | Separation Distance (mm) | MMAD (µm) | |
|---|---|---|---|---|
| | | | BDP | Budesonide |
| 60 | 0.25 | 17.5 | 2.0 | 3.1 |
| | | 9.0 | 2.1 | 3.0 |
| | | 6.0 | 2.3 | 2.9 |
| | 0.30 | 2.0 | 1.9 | 2.8 |
| 15 | 0.25 | 6.0 | 1.8 | 3.4 |

Table 13 demonstrates that the orifice distance and impingement angle influences the drug delivery characteristics. Desired characterstics may be attained by setting the orifice distance and the impingement angle.

As shown by Table 13, at 60° impingement, the MMADs became more similar as separation distance decreases, indicating that mixing does take place post-orifice.

At an impingement angle of only 15°, mixing becomes less efficient, even at a small separation distance. This smaller angle of impingement of 15° means that the plumes collide further from the orifice exits. Therefore the plume has more time to atomise and develop before impingement and mixing.

As the separation distance decreases, actuator deposition also decreases: at 15° impingement and 6.0mm separation, the actuator deposition was comparable to that of the standard actuator (6.1µg for BDP, and 5.9µg for Budesonide). However, there is an inverse relationship between actuator and induction port deposition as separation distance is changed.

### Example 14b:

In order to assess how much of the effect of dual orifice delivery is due to post-orifice mixing of the plumes originated by the contemporaneous release of the two formulations, or whether part or all is due to other factors, such as impaction on the side of the mouthpiece and induction port, a single dose of Budesonide (50µg/25µl) was delivered through the test rig in a dual orifice configuration system using only a single can.

Figure 32 and Table 14 shows that, for single-can delivery, actuator deposition is approximately doubled in comparison to dual delivery with two cans, as there is no opposite force of the second plume to direct the flow towards the mouthpiece exit.

However, actuation of a single can through this system also brings about reduced deposition on the induction port and stages 0, 1, 2 and 3, effectively reducing the MMAD from 2.9 µm (data from dual delivery with an extrafine formulation) to 2.3µm. The FPDs are almost identical (15.4µg and 15.6µg - see Table 14).

**Table 14: Dual Reservoir-Dual Orifice: Single can delivery vs. Dual can delivery**

| | Bud (Single Can) | | Bud (Delivered with BDP) | |
|---|---|---|---|---|
| Metered Dose (µg) | 59.0 | 59.4 | 59.6 | 56.6 |
| Delivered Dose (µg) | 28.1 | 32.0 | 44.7 | 44.1 |
| FPD (µg) | 15.5 | 15.3 | 16.2 | 15.0 |
| FPF (%) | 55.0 | 47.8 | 36.4 | 34.0 |
| MMAD (µm) | 2.3 | 2.3 | 2.9 | 2.9 |

### Example 15:

This example demonstrates how the PSD of one formulation is affected by simultaneous delivery of a second formulation, when a dual orifice and a single orifice configuration are used .

For the single orifice configuration, Formulations 9 and 12 (see Example 6) are fired through the test rig in a single orifice configuration, corresponding to the single orifice actuator, with a nozzle orifice diameter of 0.30mm. For the dual orifice configuration, Formulations 9 and 12 (see Example 6) are fired through the test rig in a dual orifice configuration, corresponding to the dual orifice actuator, with a nozzle orifice diameter of 0.25mm, 60° impingement angle, 6.0mm nozzle separation distance.

Comparative data were obtained by firing the BDP formulation and the Budesonide formulation respectively through a conventional actuator of a single reservoir MDI system. The nozzle orifice diameter was 0.22mm.

The delivery characteristics are shown in Fig. 33. The data obtained for the dual-reservoir MDIs with a single nozzle orifice are indicated by "single orifice". The data obtained for the dual reservoir MDIs with dual orifice configuration are indicated by "dual orifice". Comparative data are indicated by "pMDI, 0.22mm".

Delivery through a single 0.30mm diameter orifice causes the MMADs of two distinct formulations to become almost identical (2.8µm for BDP Formul 9 and 2.9µm for Budesonide Formulation 12).

Mixing does not occur to the same extent through the dual orifices. The MMAD of the BDP extrafine formulation shifts from 1.3µm (data obtained for conventional MDI) to 2.3µm, and that of the Budesonide formulation from 3.1µm (data obtained for conventional MDI) to 2.9µm. The trend is similar to that seen for single orifice delivery: the MMAD of the BDP extrafine formulation moves further towards that of the Budesonide formulation. This demonstrates that glycerol may be used to attain a significant modulation of particle size.

The comparison shows that, while mixing does take place post orifice, in order to produce identical PSDs, or very similar PSDs, from two distinct formulations, an actuator design should be selected where mixing begins in the sump.

An advantage of the dual orifice system is that PSD and MMAD can be optimized to suit any desired combination by pertinent selection of combinations of the three variables: orifice diameter, impingement angle and separation distance. It is not always required to appropriately set each one of the three parameters, but it may be sufficient to adjust only one or two of the indicated parameters to attain a desired PSD and MMAD. For illustration, the three variable may be set so as to minimize the effect of one plume on the other, in order to keep the PSDs distinct.

### Example 16:

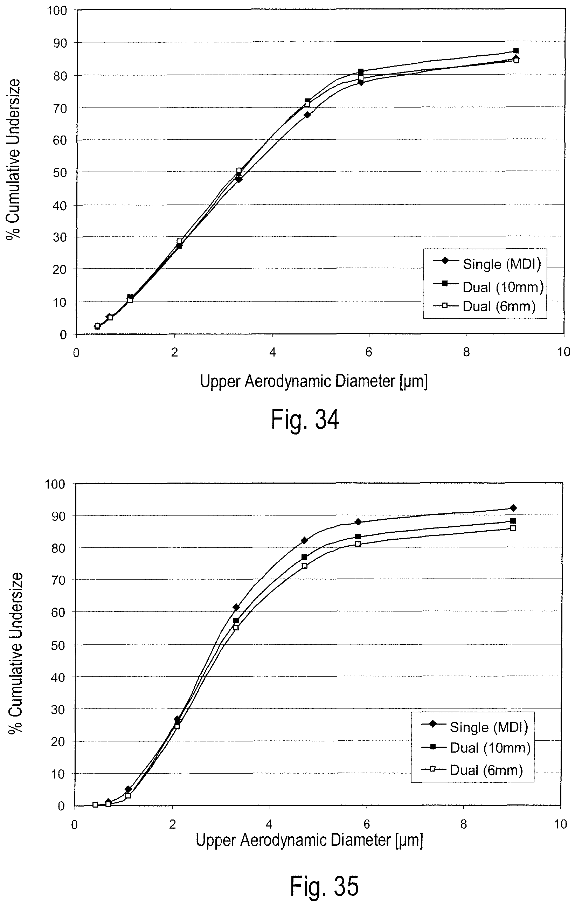

In this example, formulations were simultaneously delivered from dual reservoirs through dual orifices (Fig. 34 and Fig. 35). The two formulations used were:
Formulation 21 marketed Clenil Modulite: BDP (250µg/actuation), ethanol, glycerol, HFA 134a
Formulation 22 marketed Ventolin Evohaler: salbutamol sulphate (100µg/actuation), HFA 134a
Formulation 21 is a solution formulation, while formulation 22 is a suspension formulation.

For delivery in a dual reservoir - dual orifice configuration, the test rig was used with a dual orifice configuration. The dual reservoir - dual orifice configuration corresponds to a configuration as generally shown for the actuator as shown in Fig. 17B. Measurements were performed for two different orifice distances (6mm and 10mm). An impingement angle of 15° was chosen so as to reduce plume interaction. The orifice for delivery of formulation 21 (Clenil) had a diameter of 0.30mm. The orifice for delivery of formulation 22 (Ventolin) had a diameter of 0.50mm. Comparative data were obtained by firing the respective Formulation 21 and Formulation 22 through a conventional actuator, i.e., an actuator of a MDI having one reservoir and one orifice. For Formulation 21, the actuator had a nozzle orifice diameter of 0.30mm. For Formulation 22, the actuator had a nozzle orifice diameter of 0.50mm.

Fig. 34 shows the drug delivery characteristics for Formulation 21 (Clenil) when delivered with Formulation 22, respectively for the two different orifice separation distances of 10mm and 6mm. Fig. 35 shows the drug delivery characteristics for Formulation 22 (Ventolin) when delivered with Formulation 21, respectively for the two different orifice separation distances of 10mm and 6mm.

When the two formulations are delivered simultaneously, the PSDs of the two formulations, in particular Formulation 21 (Clenil), are closely matched with the control data obtained using a single reservoir - single orifice actuator. The FPD of the Clenil formulation decreases as the separation distance decreases (from 41.3µg at 10.0mm orifice spacing to 37.6µg at 6.0mm orifice spacing; comparative data: 55.6µg). As separation distance increases, actuator and induction port deposition increases. A similar result is observed for Ventolin: the FPD shifts from 16.8µg at 10mm orifice spacing to and 15.9µg at 6mm orifice spacing (comparative data: 35.9µg).

The MMAD does not change significantly, from 3.4µm for the comparative data (single reservoir-single orifice), to 3.3µm for dual delivery at both separation distances. The slight decrease may be explained by the effect of the extra HFA added to the plume by the Ventolin, which may boost the atomisation.

### Example 17:

In this example, the two formulations 21 and 22 (see Example 16) were simultaneously delivered from dual reservoirs through a single orifice. The nozzle orifice diameter was 0.30mm.

Comparative data were obtained in the same way as described for Example 16. I.e., MDIs having conventional single reservoir - single orifice configurations were used, with the orifice diameter being 0.30mm (Formulation 21) and 0.50mm (Formulation 22), respectively.

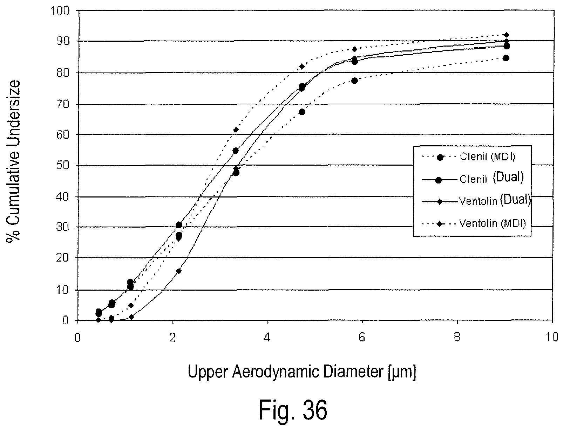

Fig. 36 and Table 15 show the delivery characteristics. As shown in Fig. 36, mixing the two formulations causes a shift in MMAD from those of the conventional MDIs, similar to the effects of mixing two solution formulations.

The MMAD of Formulation 21 (Clenil Modulite) decreases from 3.4 to 3.0µm (Table 15) due to the increase in atomisation produced by the HFA delivered by Formulation 22 (Ventolin). The FPD also increases from 56µg to 86µg. This is a similar effect to that seen in Example 12, where HFA is used to boost the atomisation of high ethanol solutions. The MMAD of the Ventolin formulation increases from 2.9 to 3.3µm, as glycerol within the Clenil formulation increases the size of the particles produced.

**Table 15: Dual Reservoir-Single Orifice Delivery of formulation 21 (Clenil Modulite) and formulation 22 (Ventolin)**

| | Clenil (conventional MDI) | Clenil (Dual reservoir) | Ventolin (Dual reservoir) | Ventolin (conventional MDI) |
|---|---|---|---|---|
| Metered Dose (µg) | 248.0 | 262.8 | 118.5 | 100.5 |
| Delivered Dose (µg) | 225.4 | 248.9 | 90.2 | 89.0 |
| FPD (µg) | 55.6 | 85.5 | 33.9 | 35.9 |
| FPF (%) | 24.7 | 34.4 | 38.1 | 40.3 |
| MMAD (µm) | 3.4 | 3.0 | 3.3 | 2.9 |
| n | 2 | 2 | 2 | 2 |

### Example 18:

In this example, a combination product (marketed Fostair, containing a formulation of a steroid (BDP) and β2 agonist (formoterol) with glycopyrrolate, which acts on muscarinic receptors) was co-administered simultaneously using the test rig in a dual reservoir-single orifice configuration with glycopyrronium bromide (GP), a muscarinic receptor antagonist. Different formulations of glycopyrronium bromide have been used.. The same configuration as in Example 17 was used, with an orifice diameter of 0.30 mm.
Combination product (marketed Fostair): BDP/FF (100/6µg/actuation), ethanol, HCI (1M), HFA134a
Formulation 23: Glycopyrronium bromide (25µg/actuation), 12% w/w ethanol, HFA 134a
Formulation 24: Glycopyrronium bromide (25µg/actuation), 12% w/w ethanol, 0.144% w/w glycerol
Formulation 25: Glycopyrronium bromide (25µg/actuation), 12% w/w ethanol, 0.144% w/w isopropyl myristate (IPM)

Comparative data for the combination product were obtained by firing a dose of the combination product using a conventional, commercial single reservoir-single orifice actuator for the combination product, having an orifice diameter of 0.30 mm.

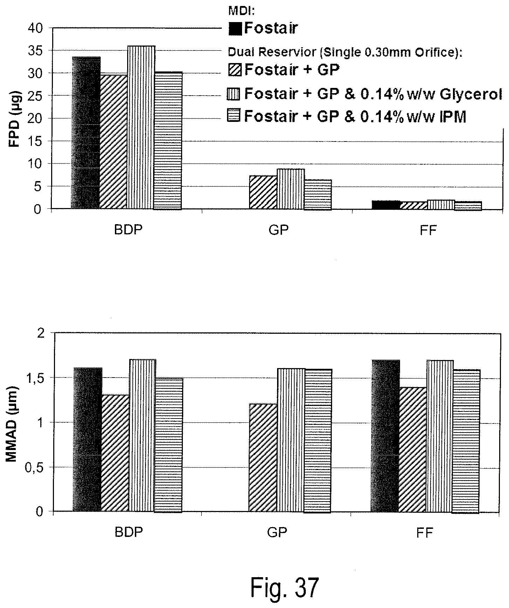

Fig. 37 shows the delivery characteristics when the combination product is fired together with one of formulations 23-25 through a single nozzle orifice. The FPDs of BDP and formoterol are comparable with the control when delivered with extrafine glycopyrrolate through the actuator of the dual reservoir-single orifice system. The addition of non-volatile additives in the glycopyrrolate formulation causes a slight increase in the MMAD of the BDP and formoterol.

The challenge of formulation is such that it is often difficult to find two active substances which are stable in the same vehicle. However, as illustrated by examples 16-18 and other examples described, and as described in detail herein, using the dual reservoir-dual orifice concept, it is possible to deliver two such drugs simultaneously. It is also possible to match two separate formulations, such as the Clenil Modulite and Ventolin, in terms of performance, thus creating a dual therapy.

### Example 19:

Two formulations were manufactured using a 16mm valve-can assemblies and delivered through the dual reservoir prototype system of Fig 14B:
BDP/ Formoterol (100µg /6µg/25µl), 12% w/w ethanol, 0.024% w/w HCl (1M), HFA 134a to 100% w/w and
Glycopyrronium bromide (12.5µg/25µl), 12% w/w ethanol, 0.019% w/w HCI (1M), HFA 134a to 100% w/w.

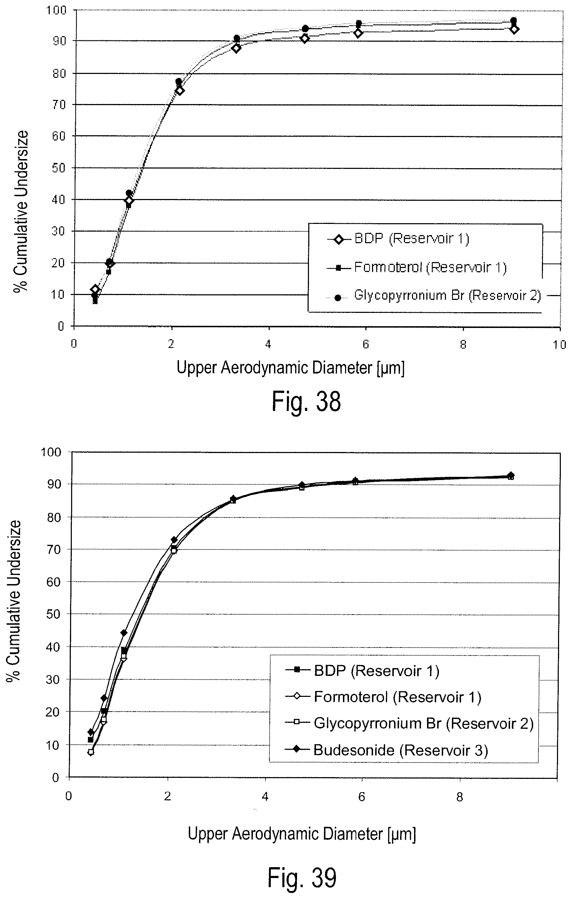

Fig. 38 shows the delivery characteristics. Fig. 38 shows that the particle size distributions of the formulations are identical, as observed when using the test rig with single orifice piece.

In Table 16 are reported the MMAD of all three drugs which is 1.3µm. This is comparable to the data collected for the same formulations using the test rig with dual reservoir-single orifice (0.30mm) configuration, as reported in Example 18 (Fig. 37). However, the FPFs for the formulations delivered through the prototype of Fig. 14B are greater (44% compared with 30-32%), as throat deposition is reduced.

**Table 16: Dual reservoir-sinle orifice prototype**

| | BDP | Formoterol | Glycopyrronium bromide |
|---|---|---|---|
| Metered Dose (µg) | 92.8 | 5.7 | 11.3 |
| Delivered Dose (µg) | 67.6 | 3.6 | 7.9 |
| FPD (µg) | 30.2 | 1.6 | 3.5 |
| FPF (%) | 44.7 | 44.3 | 44.1 |
| MMAD (µm) | 1.3 | 1.3 | 1.3 |

### Example 20:

Similar result to that obtained in Example 19 is seen in Fig. 39 for the system having three reservoirs, shown in Fig. 16B. The same BDP/Formoterol formulation as was used in Example 19 was contained in a first container of the system. The same Glycopyrronium bromide formulation as was used in Example 19 was contained in a second container of the system. A third formulation was contained in a third container, which third formulation was constituted by:
Budesonide (50µg/25µl), 12% w/w ethanol, HFA 134a to 100% w/w.

Fig. 39 illustrates the delivery characteristics.

In Table 17 it is shown that the MMADs are similar but not identical, ranging from 1.2 - 1.5µm. However, due to the fact that in the triple prototype the three valve springs may increase the force needed to depress the cans, there may be small differences in the timings of actuation of the canisters, which could affect the mixing process of the emitted aerosol clouds.

**Table 17: Triple reservoir-single orifice prototype**

| | | Glycopyrronium | | |
|---|---|---|---|---|
| | BDP | Formoterol | bromide | Budesonide |
| Metered Dose (µg) | 85.9 | 5.2 | 10.8 | 41.9 |
| Delivered Dose (µg) | 60.8 | 3.3 | 6.9 | 19.5 |
| FPD (µg) | 21.2 | 1.1 | 2.2 | 7.6 |
| FPF (%) | 34.9 | 33.9 | 32.4 | 38.8 |
| MMAD (µm) | 1.4 | 1.5 | 1.4 | 1.2 |

## Claims

1. A metered-dose inhaler (91; 131; 151), comprising:
at least one vessel (101, 102; 167), said at least one vessel (101, 102; 167) including a first reservoir (101) containing a first formulation (103) and a second reservoir (102) different from said first reservoir (101) and containing a second formulation (106), and
an actuator (92; 132-138; 152-158) for receiving said at least one vessel (101, 102),
said metered-dose inhaler (91; 131; 151) being configured to be actuable when said at least one vessel (101, 102; 167) is received by said actuator (92; 132-138; 152-158), and being configured to simultaneously deliver at least a first metered dose of said first formulation (103) from said first reservoir (101) and a second metered dose of said second formulation (106) from said second reservoir (102) when said metered-dose inhaler (91; 131; 151) is actuated, wherein said first formulation is a first pressurized aerosol formulation and said second formulation is a second pressurized aerosol formulation,
**characterized in that**
said actuator comprises a guide member (138; 158) which defines a passageway (144) for the first metered dose and another passageway (145) for the second metered dose, an exit orifice of the guide member (138; 158) being in communication with an actuator block (134; 154) having a single nozzle orifice (95) for atomizing both said first metered dose and said second metered dose upon actuation of said metered-dose inhaler (91; 131; 151);
said second formulation comprises a low volatility component and is selected such that a mass median aerodynamic diameter, MMAD, of said first formulation is modulated by the presence of the low-volatility component in the second formulation when mixing said first metered dose and said second metered dose at an interconnection point of the first passageway (144) and the second passageway (145), and prior to atomization at the nozzle orifice (95), causing a shift of the MMAD, which would be obtained by individually atomizing the first formulation, towards an MMAD which would be obtained by individually atomizing the second formulation.

2. The metered-dose inhaler (91; 131; 151) of claim 1, comprising
a first metering system (104) for metering said first metered dose and a second metering system (107) for metering said second metered dose, and
an actuating arrangement (93; 133; 153) for effecting simultaneous actuation of said first metering system (104) and said second metering system (107) upon actuation of said metered-dose inhaler, when said at least one vessel (101, 102; 167) is received by said actuator (92; 132-138; 152-158).

3. The metered-dose inhaler (91; 131; 151) of claim 1 or claim 2,
said actuator (92; 132-138; 152-158) being configured such that, when said at least one vessel (101, 102; 167) is received by said actuator (92), said first metered dose and said second metered dose are mixed prior to being delivered through a mouthpiece opening of said actuator (92; 132-138; 152-158) upon actuation of said metered-dose inhaler (91; 131; 151).

4. The metered-dose inhaler of any one of the preceding claims,
said at least one vessel (101, 102; 167) having a first valve stem (105) for supplying said first metered dose and a second valve stem (108) for supplying said second metered dose,
said actuator (92; 132-138; 152-158) having a first seat for receiving said first valve stem and a second seat for receiving said second valve stem, said nozzle orifice (95) being in communication with both said first seat and said second seat.

5. The metered-dose inhaler of any one of the preceding claims,
said at least one vessel (101, 102; 167) comprising a first vessel (101) defining said first reservoir (101) and a second vessel (102) defining said second reservoir (102) and formed separately from said first vessel (101).

6. The metered-dose inhaler of any one of the preceding claims,
said at least one vessel including a third reservoir containing a third formulation,
said metered-dose inhaler being configured, when said at least one vessel is received by said actuator, to simultaneously deliver said first metered dose of said first formulation, said second metered dose of said second formulation and a third metered dose of said third formulation from said third reservoir when said metered-dose inhaler is actuated.

## Patentansprüche

1. Inhalator mit abgemessener Dosis (91; 131; 151), umfassend:
mindestens ein Behälter (101, 102; 167), wobei der mindestens eine Behälter (101, 102; 167) ein erstes Reservoir (101), das eine erste Formulierung (103) enthält, und ein zweites Reservoir (102) , das sich von dem ersten Reservoir (101) unterscheidet und eine zweite Formulierung (106) enthält, aufweist, und
ein Bedienelement (92; 132-138; 152-158) zum Aufnehmen des mindestens einen Behälters (101, 102),
wobei der Inhalator mit abgemessener Dosis (91; 131; 151) ausgestaltet ist, um betätigbar zu sein, wenn der mindestens eine Behälter (101, 102; 167) von dem Bedienelement (92; 132-138; 152-158) aufgenommen ist, und ausgestaltet ist, um gleichzeitig mindestens eine erste abgemessene Dosis der ersten Formulierung (103) aus dem ersten Reservoir (101) und eine zweite abgemessene Dosis der zweiten Formulierung (106) aus dem zweiten Reservoir (102) abzugeben, wenn der Inhalator mit abgemessener Dosis (91; 131; 151) betätigt wird, wobei die erste Formulierung eine erste unter Druck stehende Aerosolformulierung ist und die zweite Formulierung eine zweite unter Druck stehende Aerosolformulierung ist,
**dadurch gekennzeichnet, dass**
das Bedienelement ein Führungselement (138; 158) umfasst, das einen Durchgang (144) für die erste abgemessene Dosis und einen anderen Durchgang (145) für die zweite abgemessene Dosis definiert, wobei eine Ausgangsöffnung des Führungselements (138; 158) in Verbindung mit einem Betätigungsblock (134; 154) steht, der eine einzelne Düsenöffnung (95) zum Zerstäuben sowohl der ersten abgemessenen Dosis als auch der zweiten abgemessenen Dosis bei Betätigung des Inhalators mit abgemessener Dosis (91; 131; 151) aufweist;
die zweite Formulierung eine geringflüchtige Komponente umfasst und so ausgewählt ist, dass ein massenmedialer aerodynamischer Durchmesser, MMAD, der ersten Formulierung durch das Vorhandensein der geringflüchtigen Komponente in der zweiten Formulierung moduliert wird, wenn die erste abgemessene Dosis und die zweite abgemessene Dosis an einem Verbindungspunkt des ersten Durchgangs (144) und des zweiten Durchgangs (145) gemischt werden, und vor einer Zerstäubung an der Düsenöffnung (95) eine Verschiebung der MMAD, die durch individuelles Zerstäuben der ersten Formulierung erhalten werden würde, in Richtung einer MMAD, die durch individuelles Zerstäuben der zweiten Formulierung erhalten werden würde, bewirkt.

2. Inhalator mit abgemessener Dosis (91; 131; 151) nach Anspruch 1, umfassend
ein erstes Abmesssystem (104) zum Abmessen der ersten abgemessenen Dosis und ein zweites Abmesssystem (107) zum Abmessen der zweiten abgemessenen Dosis, und
eine Betätigungsanordnung (93; 133; 153) zum Bewirken einer gleichzeitigen Betätigung des ersten Abmesssystems (104) und des zweiten Abmesssystems (107) bei Betätigung des Inhalators mit abgemessener Dosis, wenn der mindestens eine Behälter (101, 102; 167) von dem Bedienelement (92; 132-138; 152-158) aufgenommen ist.

3. Inhalator mit abgemessener Dosis (91; 131; 151) nach Anspruch 1 oder Anspruch 2,
wobei das Bedienelement (92; 132-138; 152-158) so ausgestaltet ist, dass, wenn der mindestens eine Behälter (101, 102; 167) von dem Bedienelement (92) aufgenommen ist, die erste abgemessene Dosis und die zweite abgemessene Dosis gemischt werden, bevor sie durch eine Mundstücköffnung des Bedienelements (92; 132-138; 152-158) bei Betätigung des Inhalators mit abgemessener Dosis (91; 131; 151) abgegeben werden.

4. Inhalator mit abgemessener Dosis nach einem der vorhergehenden Ansprüche,
wobei der mindestens eine Behälter (101, 102; 167) einen ersten Ventilschaft (105) zum Zuführen der ersten abgemessenen Dosis und einen zweiten Ventilschaft (108) zum Zuführen der zweiten abgemessenen Dosis aufweist,
wobei das Bedienelement (92; 132-138; 152-158) einen ersten Sitz zum Aufnehmen des ersten Ventilschafts und einen zweiten Sitz zum Aufnehmen des zweiten Ventilschafts aufweist, wobei die Düsenöffnung (95) sowohl mit dem ersten Sitz als auch mit dem zweiten Sitz in Verbindung steht.

5. Inhalator mit abgemessener Dosis nach einem der vorhergehenden Ansprüche,
wobei der mindestens eine Behälter (101, 102; 167) einen ersten Behälter (101), der das erste Reservoir (101) definiert, und einen zweiten Behälter (102), der das zweite Reservoir (102) definiert und getrennt von dem ersten Behälter (101) ausgebildet ist, umfasst.

6. Inhalator mit abgemessener Dosis nach einem der vorhergehenden Ansprüche,
wobei der mindestens eine Behälter ein drittes Reservoir aufweist, das eine dritte Formulierung enthält,
wobei der Inhalator mit abgemessener Dosis ausgestaltet ist, um, wenn der mindestens eine Behälter von dem Bedienelement aufgenommen ist, gleichzeitig die erste abgemessene Dosis der ersten Formulierung, die zweite abgemessene Dosis der zweiten Formulierung und eine dritte abgemessene Dosis der dritten Formulierung aus dem dritten Reservoir abzugeben, wenn der Inhalator mit abgemessener Dosis betätigt wird.

## Revendications

1. Aérosol-doseur (91 ; 131 ; 151), comprenant:
au moins un récipient (101, 102 ; 167), ledit au moins un récipient (101, 102 ; 167) comportant un premier réservoir (101) contenant une première formulation (103) et un deuxième réservoir (102) différent dudit premier réservoir (101) et contenant une deuxième formulation (106), et
un actionneur (92 ; 132-138 ; 152-158) permettant de recevoir ledit au moins un récipient (101, 102),
ledit aérosol-doseur (91 ; 131 ; 151) étant conçu pour pouvoir être actionné lorsque ledit au moins un récipient (101, 102 ; 167) est reçu par ledit actionneur (92 ; 132-138 ; 152-158), et étant conçu pour distribuer simultanément au moins une première dose mesurée de ladite première formulation (103) en provenance dudit premier réservoir (101) et une deuxième dose mesurée de ladite deuxième formulation (106) en provenance dudit deuxième réservoir (102) lorsque ledit aérosol-doseur (91 ; 131 ; 151) est actionné, ladite première formulation étant une première formulation d'aérosol sous pression et ladite deuxième formulation étant une deuxième formulation d'aérosol sous pression,
**caractérisé en ce que**
ledit actionneur comprend un élément de guidage (138 ; 158) qui définit un passage (144) pour la première dose mesurée et un autre passage (145) pour la deuxième dose mesurée, un orifice de sortie de l'élément de guidage (138 ; 158) étant en communication avec un bloc actionneur (134 ; 154) ayant un seul orifice de buse (95) permettant d'atomiser à la fois ladite première dose mesurée et ladite deuxième dose mesurée lors de l'actionnement dudit aérosol-doseur (91 ; 131 ; 151) ;
ladite deuxième formulation comprend un composant à faible volatilité et est sélectionnée de sorte qu'un diamètre aérodynamique moyen en masse, MMAD, de ladite première formulation est modulé par la présence du composant à faible volatilité dans la deuxième formulation lors du mélange de ladite première dose mesurée et de ladite deuxième dose mesurée au niveau d'un point d'interconnexion du premier passage (144) et du second passage (145), et avant l'atomisation au niveau de l'orifice de buse (95), amenant un décalage du MMAD, qui serait obtenu en atomisant individuellement la première formulation, vers un MMAD qui serait obtenu en atomisant individuellement la deuxième formulation.

2. Aérosol-doseur (91 ; 131 ; 151) selon la revendication 1, comprenant
un premier système de dosage (104) permettant de doser ladite première dose mesurée et un second système de dosage (107) permettant de doser ladite deuxième dose mesurée, et
un agencement d'actionnement (93 ; 133 ; 153) permettant d'effectuer un actionnement simultané dudit premier système de dosage (104) et dudit second système de dosage (107) lors de l'actionnement dudit aérosol-doseur, lorsque ledit au moins un récipient (101, 102 ; 167) est reçu par ledit actionneur (92 ; 132-138 ; 152-158).

3. Aérosol-doseur (91 ; 131 ; 151) selon la revendication 1 ou la revendication 2,
ledit actionneur (92 ; 132-138 ; 152-158) étant conçu de sorte que, lorsque ledit au moins un récipient (101, 102 ; 167) est reçu par ledit actionneur (92), ladite première dose mesurée et ladite deuxième dose mesurée sont mélangées avant d'être distribuées à travers une ouverture d'embout buccal dudit actionneur (92 ; 132-138 ; 152-158) lors de l'actionnement dudit aérosol-doseur (91 ; 131 ; 151).

4. Aérosol-doseur selon l'une quelconque des revendications précédentes,
ledit au moins un récipient (101, 102 ; 167) ayant une première tige de soupape (105) permettant de fournir ladite première dose mesurée et une seconde tige de soupape (108) permettant de fournir ladite deuxième dose mesurée,
ledit actionneur (92 ; 132-138 ; 152-158) ayant un premier siège permettant de recevoir ladite première tige de soupape et un second siège permettant de recevoir ladite seconde tige de soupape, ledit orifice de buse (95) étant en communication à la fois avec ledit premier siège et avec ledit second siège.

5. Aérosol-doseur selon l'une quelconque des revendications précédentes,
ledit au moins un récipient (101, 102 ; 167) comprenant un premier récipient (101) définissant ledit premier réservoir (101) et un second récipient (102) définissant ledit deuxième réservoir (102) et formé séparément dudit premier récipient (101).

6. Aérosol-doseur selon l'une quelconque des revendications précédentes,
ledit au moins un récipient comportant un troisième réservoir contenant une troisième formulation,
ledit aérosol-doseur étant conçu, lorsque ledit au moins un récipient est reçu par ledit actionneur, pour distribuer simultanément ladite première dose mesurée de ladite première formulation, ladite deuxième dose mesurée de ladite deuxième formulation et une troisième dose mesurée de ladite troisième formulation en provenance dudit troisième réservoir lorsque ledit aérosol-doseur est actionné.
